(19) Europäisches Patentamt
European Patent Office
Office européen des brevets

(11) Veröffentlichungsnummer: **0 055 693**
**B1**

(12) **EUROPÄISCHE PATENTSCHRIFT**

(45) Veröffentlichungstag der Patentschrift: **20.01.88**

(51) Int. Cl.⁴: **C 07 D 239/30,** A 01 N 25/32, C 07 D 405/10

(21) Anmeldenummer: **81810505.8**

(22) Anmeldetag: **17.12.81**

(54) **Verwendung von Phenylpyrimidinen als Gegenmittel zum Schützen von Kulturpflanzen vor durch Herbizide verursachte phytotoxische Schäden.**

(30) Priorität: **23.12.80 CH 9522/80**
**08.04.81 CH 2363/81**

(43) Veröffentlichungstag der Anmeldung:
**07.07.82 Patentblatt 82/27**

(45) Bekanntmachung des Hinweises auf die Patenterteilung:
**20.01.88 Patentblatt 88/03**

(84) Benannte Vertragsstaaten:
**AT BE CH DE FR GB IT LI NL**

(56) Entgegenhaltungen:
**EP-A-0 020 298**
**DE-A-2 202 820**
**FR-A- 672 216**
**FR-A-1 469 787**
**FR-A-2 182 994**
**US-A-3 498 984**
**US-A-3 503 976**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG**
**Klybeckstrasse 141**
**CH-4002 Basel (CH)**

(72) Erfinder: **Burdeska, Kurt, Dr.**
**Paracelsusstrasse 64**
**CH-4058 Basel (CH)**
Erfinder: **Kabas, Guglielmo, Dr.**
**Im Egg 36**
**CH-4147 Aesch (CH)**
Erfinder: **Brunner, Hans-Georg, Dr.**
**Wannenstrasse 14**
**CH-4415 Lausen (CH)**
Erfinder: **Föry, Werner, Dr.**
**Benkenstrasse 65**
**CH-4054 Basel (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Description**

Die vorliegende Erfindung betrifft ein Verfahren sowie ein Mittel zum Schützen von Kulturpflanzen vor der phytotoxischen Wirkung von Herbiziden. Dabei werden Phenylpyrimidine der unterstehenden Formel I gleichzeitig oder in kurzen Folge mit dem Herbizid den Kulturpflanzungen appliziert oder es wird ein Mittel, welches neben dem Herbizid noch ein Phenylpyrimidin der Formel I enthält, angewendet. Die Erfindung betrifft auch die Mittel, welche Phenylpyrimidine der Formel I enthalten.

Die Phenylpyrimidine entsprechen der Formel I

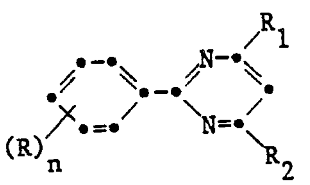

(I),

worin

n Null oder eine Zahl von 1 bis 3

R Halogen, Nitro, Cyan, $-XR_4$, $-NR_5R_6$, $-COR_3$, $-COOR_3$, $CONR_5R_6$, $-CS-NR_5R_6$, $-CH_2PO(OC_2H_5)_2$, $-SO_2-NR_6R_7$, $-SO_3H$, oder eine unsubstituierte oder durch Halogen, $-XR_4$, $-NR_5R_6$, $-COR_3$, $-COOR_3$, $-COR_5R_6$ oder Cyan substituierte $C_1-C_6$-Alkyl- oder $C_3-C_6$-Cycloalkylgruppe oder eine unsubstituierte oder durch Halogen oder $-XR_3$ substituierte $C_5-C_6$-Alkenyl-, $C_3-C_6$-Cycloalkenyl oder $C_3-C_6$-Alkinylgruppe,

$R_1$ und $R_2$ jeweils Halogen,

$R_3$ Wasserstoff, eine unsubstituierte oder durch Halogen, $-COR_3$, $-CONR_5R_6$, Hydroxy, $C_1-C_6$-Alkoxy oder $-NR_5R_6$ substituierte $C_1-C_6$-Alkyl- oder $C_3-C_6$-Alkenyl- oder $C_3-C_6$-Alkinylgruppe,

$R_4$ dasselbe wie $R_3$, ausserdem $C_1-C_6$-Alkylcarbonyl, $C_3-C_6$-Alkenylcarbonyl, $C_3-C_6$-Alkinylcarbonyl,

$R_5$ und $R_6$ unabhängig voneinander je Wasserstoff, $C_3-C_6$-Alkenyl, $C_3-C_6$-Alkinyl, $C_3-C_6$-Cycloalkyl, oder unsubstituiertes oder durch $-COR_3$ substituiertes $C_1-C_6$-Alkyl, oder einer der Reste $R_5$ und $R_6$ bedeutet eine Gruppe $-OR_4$ oder $-COR_3$, $-COOR_3$, $CONR_5R_6$ und

X Sauerstoff, Schwefel, $-SO-$ oder $-SO_2-$ bedeuten.

Der Ausdruck Alkyl allein oder als Teil eines Substituenten umfasst verzweigte oder unverzweigte Alkylgruppen, welche die angegebene Anzahl Kohlenstoffatome enthalten. Beispiele sind Methyl, Aethyl, Propyl, Isopropyl, Butyl, Isobutyl, sec.-Butyl, tert.-Butyl, sowie die höheren Homologen Amyl, Isoamyl, Hexyl, samt ihren Isomeren. Entsprechend können die Alkenyl- und Alkinylgruppen geradkettig oder verzweigt sein. Cycloalkylgruppen sind Cyclopropyl, Cyclobutyl, Cyclopentyl und Cyclohexyl.

Die Phenylpyrimidine der Formel I eignen sich hervorragend, Kulturpflanzen wie Kulturhirse, Reis, Mais, Getreidearten (Weizen, Roggen, Gerste, Hafer), Baumwolle, Zuckerrüben, Zuckerrohr, Soja etc. vor dem Angriff von pflanzenaggressiven Agrarchemikalien, insbesondere von Herbiziden verschiedenster Stoffklassen, wie Triazinen, Phenylharmstoffderivaten, Carbamaten, Thiolcarbamaten, Halogenacetaniliden, Halogenphenoxyessigsäureestern, substituierte Phenoxyphenoxyessigsäureestern und -propionsäureestern, substituierten Pyridinoxyphenoxy-essigsäureestern und -propionsäureestern, Benzoesäurederivaten usw. zu schützen, sofern diese nicht oder nicht genügend selektiv wirken, also neben den zu bekämpfenden Unkräutern auch die Kulturpflanzen mehr oder weniger schädigen. Die Erfindung betrifft auch Mittel, welche diese Phenylpyrimidine der Formel I zusammen mit Herbiziden enthalten.

Unter den Verbindungen der Formel I haben sich vor allem diejenigen bewährt, in denen

n Null oder eine Zahl von 1 bis 3

R Halogen, Cyan, Nitro, oder Mercapto Hydroxyl, eine $C_1-C_6$-Alkyl-, $C_1-C_6$-Alkoxy- oder $C_1-C_6$-Alkylthiogruppe, die unsubstituiert oder durch Halogen substituiert ist,

eine $C_2-C_6$-Alkenyl-, $C_2-C_6$-Alkinyl oder $C_3-C_6$-Cycloalkylgruppe, der Phenyl-, Carboxyl- oder Formylrest oder Aminorest, Carbamoylrest, eine $C_1-C_6$-Alkylcarbonyl-, $C_1-C_6$-Alkylamino-, Di($C_1-C_6$-Alkyl)amino-, Di($C_1-C_6$-Alkyl)carbamoylgruppe oder zwei benachbarte Reste bilden eine $C_1-C_6$-Alkylendioxygruppe und

$R_1$ und $R_2$ unabhängig voneinander Halogen bedeuten.

Unter diesen Verbindungen wirken diejenigen am besten, in denen $R_1$ und $R_2$ je ein Chloratom bedeuten, und worin sich R in der meta- oder para-Stellung in Bezug auf den Pyrimidinring befinden.

Als Gegenmittel oder Antidote sind schon verschiedene Stoffe vorgeschlagen worden, welche befähigt sind, die schädigende Wirkung eines Herbizides auf die Kulturpflanze spezifisch zu antagonisieren, d.h. die Kulturpflanze zu schützen, ohne dabei die Herbizidwirkung auf die zu bekämpfenden Unkräuter merkliche zu beeinflussen. Dabei kann ein solches Gegenmittel, auch Safener genannt, je nach seinen Eigenschaften, zur Vorbehandlung des Saatgutes der Kulturpflanze (Beizung des Samens oder der Stecklinge) oder vor der Saat in die Saatfurchen oder als Tankmischung zusammen mit dem Herbizid vor oder nach dem Auflaufen der Pflanzen verwendet werden.

So beschreibt die GB—A—1 277 557 die Behandlung von Samen bzw. Sprösslingen von Weizen und Hirse mit gewissen Oxamsäureestern und Amiden vor dem Angriff durch N-Methoxymethyl-2',6'-diäthylchloracetanilid (Alachlor). In andere Literaturstellen (DE—A—1 952 910, DE—A—2 245 471, FR—PS—A—2 021 611) werden Gegenmittel zur Beandlung von Getreide, Mais- und Reis-Samen zum Schutz gegen den Angriff herbizider Thiolcarbamate vorgeschlagen. In der DE—C—1 576 676 und der US—A—3 131 509 werden Hydroxy-amino-acetanilide und Hydantoine für den Schutz von Getreidesamen gegenüber Carbamaten wie IPC, CIPC etc. vorgeschlagen. In der weiteren Entwirklung haben sich alle diese Präparate jedoch als ungenügend erwiesen.

Ueberraschenderweise besitzen Phenylpyrimidine der Formel I die Eigenschaft, Kulturpflanzen vor dem Angriff pflanzeaggressiver Agrarchemikalien zu schützen, insbesondere vor Herbiziden der verschiedensten Stoffklassen, wie beisteilsweise Chloracetanilide, Chloracetamide, Carbamate und Thiocarbamate, Diphenyläther und Nitrodiphenyläther, Benzoesäurederivate, Triazine und Triazinone, Phenylharnstoffe, Nitroaniline, Oxdiazolone, Pyridyloxyphenoxyderivate, Phosphate und Pyrazole, sofern diese nicht oder ungenügend kulturentolerant sind.

Vorzugsweise werden die Herbizide der Klassen Chloracetanilide, Chloracetamide, Thiocarbamate, Phosphate durch die erfindungsgemässen Phenylpyridine geschützt.

Ein solches Gegenmittel oder Antidote der Formel I kann je nach Anwendungszweck zur Vorbehandlung des Saatgutes der Kulturpflanze (Beizung des Samens oder der Stecklinge) eingesetzt oder vor oder nach der Saat in den Erdboden gegeben werden oder aber für sich allein oder zusammen mit dem Herbizid or oder nach dem Auflaufen der Pflanzen appliziert werden. Die Behandlung der Pflanze oder des Saatgutes mit dem Antidote kann daher grundsätzlich unabhängig vom Zeitpunkt der Applikation der phytotoxischen Chemikalie erfolgen. Sie kann jedoch auch gleichzeitig durchgeführt werden (Tankmischung). Vorauflauf-Behandlung schliesst sowohl die Behandlung der Anbaufläche vor der Aussaat (ppi = "pre plant incorporation") als auch die Behandlung der angesäten, aber noch nicht bewachsenen Anbauflächen ein.

Die Aufwandmengen des Antidotes im Verhältnis zum Herbizid richten sich weitgehend nach der Anwendungsart. Sofern eine Feldbehandlung vorgenommen wird, entweder als Tankmischung oder bei getrennter Applikation von Herbizid und Gegenmittel, verhalten sich die Mengen von Gegenmittel zu Herbizid in der Regel wie 1:100 bis 10:1, bevorzugt wird jedoch der Bereich 1:5 bis 8:1, besonders 1:1.

Bei Samenbeizung und ähnlich gezielten Schutzmassnahmen werden jedoch weit geringere Mengen Gegenmittel im Vergleich mit den z.B. später pro Hektar Anbaufläche verwendeten Herbizidmengen benötigt. Bei der Samenbeizung werden üblicherweise pro kg Samen 0,1—10 g Gegenmittel benötigt, die bevorzugte Menge liegt zwischen 1 und 2 Gramm. Falls das Gegenmittel kurz vor der Aussaat durch Samenquellung appliziert werden soll, werden z.B. Gegenmittel-Lösungen, welche den Wirkstoff in einer Konzentration von 1—10 000 ppm enthalten, bevorzugt 100—10000 ppm, verwendet.

In der Regel folgen sich protektive Massnahmen wie Samenbeizung mit einem Gegenmittel der Formel I und mögliche spätere Feldbehandlung mit Agrarchemikalien in zwitlich grösserem Abstand. Vorbehandeltes Saat- und Pflanzengut kann später in Landwirtschaft, Gartenbau und Forstwirtschaft mit unterschiedlichen Chemikalien in Berührung kommen, Die Erfindung bezieht sich daher auch auf kulturpflanzenprotektive Mittel, die als Wirkstoff ein Gegenmittel der Formel I zusamen mit üblichen Trägerstoffen enthalten. Solche Mittel können gegebenenfalls zusätzlich mit jenen Agrarchemikalien gemischt sein, vor deren Einfluss die Kulturpflanze geschützt werden soll, z.B. mit einem Herbizid.

Als Kulturpflanzen gelten im Rahmen vorliegender Erfindung alle Pflanzen, die in irgendeiner Form Etragsstoffe produzieren (Samen, Wurzeln, Stengel, Knollen, Blätter, Blüten, Inhaltsstoffe wie Oele, Zucker, Stärke, Eiweiss etc.) und zu diesem Zweck angebaut und gehegt werden. Zu diesen Pflanzen gehören beispielsweise sämtliche Getreidearten, Weizen, Roggen, Gerste, Hafer, daneben vor allem Reise, Kulturhirse, Mais, aber auch Baumwolle, Zuckerrüben, Zuckerrohr, Soja, Bohnen, Erbsen.

Das Gegenmittel soll überall dort eingesetzt werden, wo eine Kulturpflanze vor der Phytotoxizität einer Chemikalie geschütz werden soll.

Als Herbizide, vor deren Wirkung es die Kulturpflanzen zu schützen gilt, seien beispielsweise folgende genannt:

*Chloracetanilide:* 2 - Chlor - 2',6' - diäthyl - N - (2'' - propyloxyäthyl)acetanilid ("Propalochlor"), 2 - Chlor - 6' - äthyl - N - (2'' - methoxy - 1'' - methyläthyl) - acet - o - toluidid ("Metolachlor"), 2 - Chlor - 2',6' - diäthyl - N - (butoxymethyl)acetanilid ("Butachlor"), 2 - Chlor - 6' - äthyl - N - (äthoxymethyl)-acet - o - toluidid ("Acetochlor"), 2 - Chlor - 6' - äthyl - N - (2'' - propoxy - 1'' - methyläthyl)acet - o - toluidid, 2 - Chlor - 2',6' - dimethyl - N - (2'' - methoxy - 1'' - methyläthyl)acetanilid, 2 - Chlor - 2',6' - dimethyl - N - (2'' - methoxyäthyl)acetanilid ("Dimethachlor"), 2 - Chlor - 2',6' - diäthyl - N - (pyrazol - 1 - ylmethyl)acetanilid, 2 - Chlor - 6' - äthyl - N - (pyrazol - 1 - ylmethyl)acet - o - toluidid, 2 - Chlor - 6' - äthyl - N - (3,5 - dimethyl - pyrazol - 1 - ylmethyl)acet - o - toluidid, 2 - Chlor - 6' - äthyl - N - (2'' - butoxy - 1'' - methyläthyl)acet - o - toluidid ("Metazolachlor"), 2 - Chlor - 6' - äthyl - N - (2'' - butoxyl - 1'' - (methyläthyl)acet - o - toluidid, 2 - Chlor - 2' - trimethylsilyl - N - (butoxymethyl)-acetanilid, 2 - Chlor - 2',6' - diäthyl - N - (methoxymethyl)acetanilid ("alachler") und 2 - Chlor - 2',6' - diäthyl - N - (äthoxycarbonylmethyl)acetanilid.

*Chloracetamide:* N - [1 - Isopropyl - 2 - methylpropen - 1 - yl - (1)] - N - (2' - methoxyäthyl) - chloracetamid.

*Carbamate und Thiocarbamate:* N(3′,4′ - Dichlorphenyl) - propionanilid ("propanil"), S - 4 - Chlorbenzyl - diäthyl - thiocarbamat ("Thiobencarb"), S - Aethyl - N,N - hexamethylen - thiocarbamat ("Molinate"), S - Aethyl - dipropyl - thiocarbamat ("EPTC"), N,N - di - sec.Butyl - S - benzyl - thiocarbamat (Drepamon), S - (2,3 - Dichlorallyl) - di - isopropylthiocarbamat und S(2,3,3 - Trichlorallyl) - di - isopropylthiocarbamat ("Di - und Tri - allate"), 1 - (Propyl - thiocarbonyl) - decahydro - chinaldin, S - 4 - Benzyldiathylthiocarbamat sowie entsprechende Sulfinylcarbamate.

*Diphenyläther und Nitrodiphenyläther:* 2,4 - Dichlorphenyl - 4′ - nitrophenyläther ("Nitrofen"), 2 - Chlor - 1 - (3′ - äthoxy - 4′ - nitrophenoxy) - 4 - trifluormethyl - benzol ("Oxyfluorfen"), 2′,4′ - Dichlorphenyl - 3 - methoxy - 4 - nitrophenyl - äther ("Chlormethoxinyl"), 2 - [4′ - (2″,4″ - Dichlorphenoxy) - phenoxy)propionsäure - methylester, N - (2′ - Methoxyäthyl) - 2 - [5′ - (2″ - chlor - 4″ - trifluormethylphenoxy) - phenoxy] - propionsäureamid.

*Benzosäurederivate:* Methyl - 5 - (2′,4′ - dichlorphenoxy) - 2 - nitrobenzoat ("Bifenox"), 5 - (2′ - Chlor - 4′ - trifluormethylphenoxy) - 2 - nitrobenzoesäure ("Acrifluorfen"), 2,6 - Dichlorbenzonitril ("Dichlobenil").

*Triazine und Triazinone:* 2,4 - Bis(isopropylamino) - 6 - methylthio - 1,3,5 - triazin ("Prometryn"), 2,4 - bis(äthylamino) - 6 - methylthio - 1,3,5 - trazin ("Simetryn"), 2 - (1′,2′ - Dimethylpropylamino) - 4 - äthylamino - 6 - methylthio - 1,3,5 - triazin ("Dimethametryn"), 4 - Amino - 6 - tert.butyl - 4,5 - dihydro - 3 - methylthio - 1,2,4 - triazin - 5 - on ("Metribuzin").

*Phenylharnstoffe:* N - (3′ - Isopropylphenyl) - N′,N′ - dimethyl - harnstoff ("Isoproturon"), N - (3′,4′ - Dimethylbenzyl) - N′ - 4 - tolyl - harnstoff ("Dimuron"), N - (3′ - Chlor - 4′ - isopropylphenyl) - N′,N′ - (3 - methyl - pentamethylen - 1,5 - yl) - harnstoff.

*Nitroaniline:* 2,6 - Dinitro - N,N - dipropyl - 4 - trifluormethylanilin ("Trifluralin"), N - (1′ - Aethylpropyl) - 2,6 - dinitro - 3,4 - xylidin ("Pendimethalin").

*Oxadiazolone:* 5 - tert. - Butyl - 3 - (2′,4′ - dichlor - 5′ - isopropoxyphenyl) - 1,3,4 - oxadiazol - 2 - on ("Oxidiazon").

*Pyridyloxyphenoxyderivate:* 2 - [4′ - (3″,5″ - Dichlorpyridyl - 2″ - oxy)phenoxy]propionsäure - (2 - propinyl)ester.

*Phosphate:* S - 2 - Methylpiperidino - carbonylmethyl - O,O - dipropyl - phosphorodithioat ("Piperophos").

*Pyrazole:* 1,3 - Dimethyl - 4 - (2′,4′ - dichlorbenzoyl) - 5 - (4′ - tolylsulfonyloxy) - pyrazol.

Das als Gegenmittel wirkende Phenylpyrimidin der Formel I kann wahlweise vor oder nach Applikation der Agrarchemikalie oder auch gleichzeitig mit der Agrarchemikalie angewendet werden.

Viele erfindungsgemässe Phenylpyrimidine sind neu, andere sind bekannte Verbindungen. Phenylpyrimidine werden als Zwischenprodukte in der Farbstoffherstellung benutzt, siehe z.B. GB—A—1 502 912 oder die EP—A—20 298 und EP—A—31 796. Man trifft sie ebenfalls als Zwischenprodukte von pharmakologisch aktiven Verbindungen, s. J. Med. Chem. *1978* (21), p. 123—126 oder sonst in der chemischen Literatur Bull. Soc. chem. Jap. 44 (8), p. 2182—2185 (1971). Darüber hinaus sind 2-Phenyl-4,6-dihalogenpyrimidine, z.B. in den folgenden Publikationen beschrieben:US—A—3422893, Ang. Chemie *89* (11) 816/817 (1977), J. Chem. Soc. *1952* 330—332, J. Chem. Soc. *1965* 5468, J. prakt. chem. *312* (1979) 495, US—A—3940395, US—A—3498984, FR—A—1469787, US—A—3442898, DE—A—2202820, US—A—3503976, GB—A—309033, FR—672216, J. Med. Chem. *11* (6) 1968 1227/8, BE—A—644765, DE—A—2261755, DE—A—2255525, DE—A—2258609, Farm Zh (Kiew) *76* (5) 30—36, Yakugaku Zasshi (Japan) *88* 1179 (1968).

In keiner Literaturstelle wird auf eine mögliche Verwendung von Phenylpyrimidinen in der Landwirtschaft, als Gegenmittel zum Schützen von Kulturpflanzen vor der phytotoxischen Wirkung von Herbiziden hingewiesen.

Die Phenylpyrimimdine der Formel I können durch bekannte Synthesewege hergestellt werden. Der 2-Phenylpyrimidinring wird z.B. durch Kondensation eines Phenylamidins mit einem Malonsäurederivat hergestellt.

Die erfindungsgemässen 2-Phenylpyrimidine werden dadurch erhalten, dass man in alkoholischbasischer Lösung ein Phenylamidin mit einem Malonsäuredialkylester kondensiert.

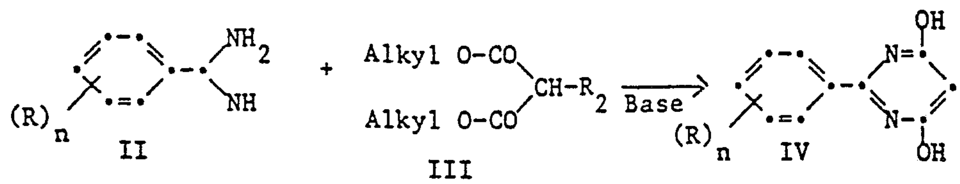


und dann gewünschtenfalls am erhaltenen 2-Phenyl-4,6-dihydroxy-pyrimidin der Formel IV die Hydroxylgruppen mittels Halogenierungsmitteln (Phosphoroxychlorid, Phosphoroxybromid, Sulfurylchlorid, Bromsuccinimid etc.) durch Halogenatome und auch diese gewünschtenfalls durch weitere Reste $R_1$ und $R_2$ ersetzt.

Die Halogenatome in den Positionen 4 und 6 des Pyrimidinringes wiederum lassen sich ihrerseits in bekannter Weise durch Alkohole, Merkaptane oder Amine ersetzen.

Siehe dazu z.B. J. Chem. Soc. *1965*, S. 5467—5473, J. prakt. Chem. *312* (1970), S. 494—506. J. chem. Soc. Perkin Trans 1 *1977*, S. 2285—6.

Phenylpyrimidine, in denen $R_1$ einen Alkyl- oder Phenylrest bedeuten soll, werden beispielsweise durch Kondensation eines Phenylamidins mit einem Alkylester einer Acetessigsäure erhalten.

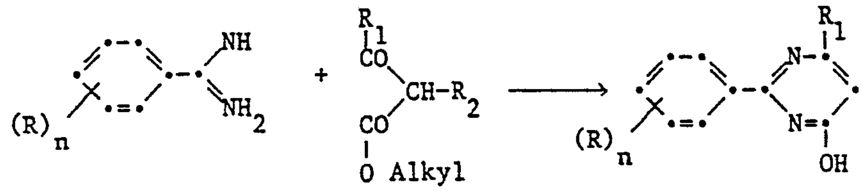

Auch hier kann die —OH Gruppe dann in bekannter Weise durch ein Halogenatom und dieses weiter durch einen Alkohol-, thiol- oder ein Aminrest ersetzt werden.

Ferner gelingt es z.B. auch 2-Phenyl-4,6-dichlorpyrimidin und 2-Phenyl-4-chlor-6-hydroxypyrimidine herzustellen durch Umsetzung von Chlorbenzyliden-carbamoyl-chloride mit einem aliphatischen Nitril in Gegenwart von Chlorwasserstoff

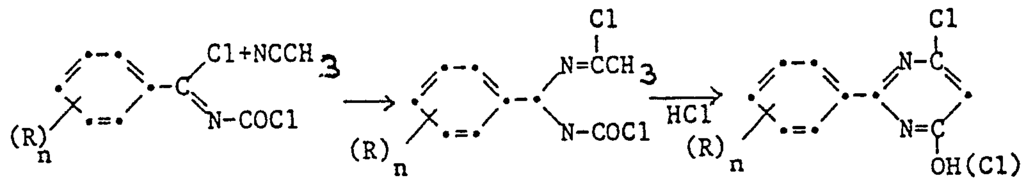


Siehe dazu Bull. Soc. Chem. Japan *44* (1971), S. 2182—2185. 2-Phenyl-4,6-dichlorpyrimidin lässt sich beispielsweise gemäss Ang. Chemie *89* (1977), S. 816—817 beispielsweise durch Kondensation von einem N-Phenylcyanamid und einem N,N-Dialkylamid in $POCl_3$ bei 100° erhalten.

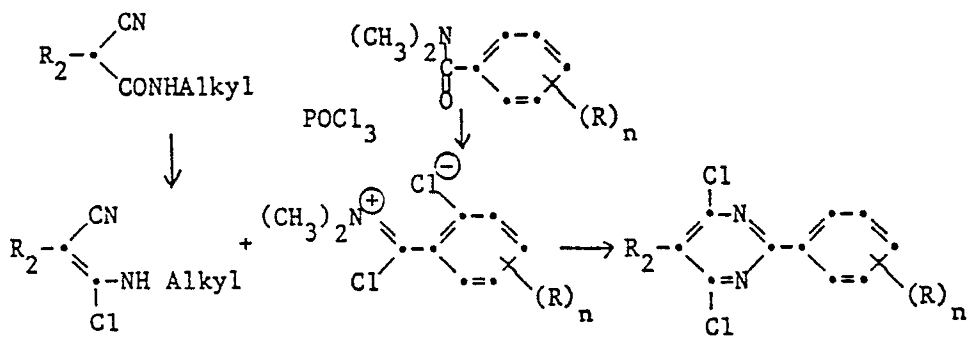


In den obigen Formeln haben R, $R_2$ und n die in Formel I gegebene Bedeutung.

Die Synthese solcher Verbindungen oder der Austausch von Resten $R_1$, $R_2$ und $R_3$ durch andere in der Definition gegebene Substituenten sind an sich bekannte Vorgänge. Für die Herstellung dieser Verbindungen verweisen wir auf die Beispiele oder auf die Fachliteratur. Siehe dazu auch "The Chemistry of Heterocyclic compounds" *16* Interscience Publishers, New York *1962* Seiten 119 ff.

Die Verbindungen der Formel I können für sich allein oder zusammen mit den zu antagonisierenden Wirkstoffen verwendet werden.

Dabei werden Verbindungen der Formel I in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und glykole sowie deren Aether und Ester, wie Aethanol, Aethylenglykol, Aethylenglykolmonomethyl; oder äthyläther, Ketone wie Cyclohexanon, stark polare Lösungsmittel wie N-Methyl-2-pyrrolidon, Dimethylsulfoxid ode Dimethylformamid, sowie gegebenenfalls epoxydierte Pflanzenöle woe epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkus, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendit werden.

Als oberflächenaktive Verbindungen kommen je nach der Art des zu formulierenden Wirkstoffes der Formel I nichtionogene, kation; und/oder anionaktive Tenside mit guten Emulgier-, Dispergier- und Netzeigenschaften in Betracht. Unter Tensiden sind auch Tensidgemische zu verstehen.

Geeignete aninische Tenside können sowohl sog. wasserlösliche Seifen wie wasserlösliche synthetische oberflächenaktive Verbindungen sein.

As Seifen eignen sich die Alkali-, Erdalkali- oder gegebenenfalls substituierten Ammoniumsalz von höheren Fettsäuren ($C_{10}$—$C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäure-methyl-taurinsalz zu erwähnen.

Häufiger werden jedoch sog. synthetisch Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substitiuerte Ammoniumsalze vir und weise einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hier her gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit 8—22 C-Atomen. Alkylarylsulfonate sind z.B. die Na-Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate, wie z.B. Salze des Phosphorsäureesters eines p-Nonylphenol-(4-14)-Aethylenoxid-Adduktes in Frage.

Als nichtionische Tenside kommen in erster Linie Polyglykolätherderivate von aliphatischen oder cycloaliphatischen Alkoholen, gesättigten oder ungesättigten Fettsäuren und Alkkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidpaddukte an Polypropylenglykol, Aethylendiaminopolypropylenglykol und Alkylpolypropylenglykol mit 1 bis 10 Kohlenstoffatomen in der Alkylkette., Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinussölpolyglykoläther, Polypropylen-Polyäthylenoxyaddukte, Tributylphenoxypolyäthoxyäthanol, Polyäthylenglykol und Octylphenoxypolyäthoxyäthanol erwähnt.

Ferner konnen auch Fettsäureester von Polyoxyäthylensorbitan wie das Polyoxyäthylensorbitantrioleat in Betracht.

Bei den kationischen Tensiden handelt es sich vor allem um quartäre Ammoniumsalze, welche als N-Substituenten mindestens winen Alkylrest mit 8 bis 22 C-Atomen enthalten und als weitere Substituenten niedrige, gegebenenfalls halogneierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)äthylammoniumbromid.

Die in Der Formulierungstechnik gebräuchlichen Tenside sind u.a. in folgenden Publikationen beschrieben:

"Mc Cutcheon's Detergents and Emulsifiers Annual" MC Publishing Corp., Ringwood, New Jersey, 1979. Sisely and Wood, "Encyclopedia of Surface Active Agents", Chemical Publishing Co., Inc. New York, 1964.

Die Pestiziden Zubereitungen enthalten in der Regel 0,1 bis 99%, insbesondere 0,1 bis 95%, Wirkstoff der Formel I, 1 bis 99% eines festen oder flüssigen Zusatzstoffes und 0 bis 25%, insbesondere 0,1 bis 25%, eines Tensides.

Während als Handelsware eher konzentrierte Mittel bevorzugt werden, verwendet der Endverbraucher in der Regel verdünnte Mittel.

Die Mittel Können auch weitere Zusätze wie Stabilisatoren, Entschäumer, Viskositätsregulatoren, Bindemittel, Haftmittel, sowie Dünger oder andere Wirkstoffe zur Erzielung spezieller Effekte enthalten.

In den nachfolgenden Beispielen sind die Temperaturen in Celsiusgraden angegeben, Prozente und Angaben von "Teilen" beziehen sich auf das Gewicht.

*Herstellungsbeispiele:*

Beispiel 1

2-para-Tolyl-4,6-dihydroxy-pyrimidin

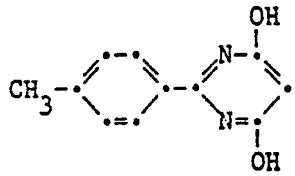

Zwischenprodukt fur Verbindung No. 2

102,3 g p-Tolylamidinhydrochlorid und 99,3 g Malonsäurediäthylester werden in 520 ml wasserfreiem Aethanol angeschlämmt. Unter gutem Rühren und Kühlung lässt man nun 323,7 g einer 30%igen Natriummethylatlösung einfliessen. Dansch wird zum Rückfluss erhitzt und 4 bis 5 Stunden am Rüssfluss gerührt. Nach Abdestillation des Lösungsmittels wird der Rückstand in 1000 ml Wasser aufgenommen, auf 80°C erhitzt und die etwas trübe Lösung über Kieselsäure filtriert. Nach dem Abkühlen wird mit 15%iger Salzsäure angesäuert. Der dicke Kristallbrei wird abfiltriert, mit Wasser gewaschen und bei 100°C getrocknet. Man erhält 100—110 g 2-para-Tolyo-4,6-dihydroxypyrimidin, Schmelzpunkt 314°C (Zersetzung).

Beispiel 2

2-para-Tolyl-4,6-dichlor pyrimidin

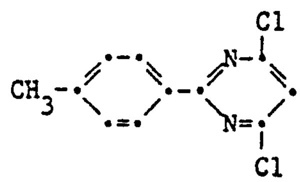

Verbindung No. 2

72,6 g der Dihydroxy-Verbindung werden mit 72,6 g N,N-Dimethylanilin und 363 g Phosphoroxychlorid zum Sieden erhitz und eine Stunde am Rückfluss gerührt. Nach Abdestillation des überschüssigen Phosphoroxychlorids wird das zurückbleibende Produkt, zur Entfernung des noch anhaftenden Phosphoroxychlorids, mit Eiswasser behandelt, danach mit Eiswasser fein gemahlen, abfiltriert, mit Eiswasser gewaschen und bei 40—50°C im Vakuum getrocknet. Man erhält so 85,9 g 2-para-Tolyl-4,6-dichloro-pyrimidin vom Schmelzpunkt 86—87°C.

Beispiel 3

2-para-Chlorphenyl-4,6-dihydroxy-pyrimidin

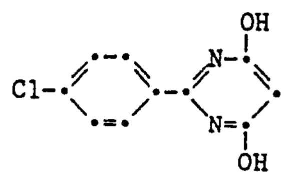

Zwischenprodukt fur Verbindung No. 3

Zu einer Suspension von 38,2 g 4-Chlorbenzamidin-Hydrochlorid und 33,6 g Malonsäurediäthylester in 175 ml Methanol gibt man innerhalb 10 Minuten 108 g 30%ige Natriummethylat/Methanol-Lösung und kocht anschliessend das Ganze während 5 Stunden am Rückfluss. Dan wird das Lösungsmittel am Rotationsverdampfer abdestilliert und der Rückstand in 1000 ml heissem Wasser aufgenommen und filtriert. Das Filtrat wird dann auf pH 1 angesäuert, der ausgefallene Niederschag wird abfiltriert und im Vakuum bei 80°C getrocknet. Man erhält so 44 g 2-para-Chlorphenyl-4,6-dihydroxypyrimidin mit einem Schmelzpunkt von 333°C (Zersetzung).

Beispiel 4

2-para-Chlorphenyl-4,6-dichlor-pyrimidin

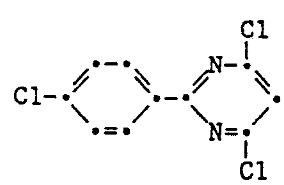

Verbindung No. 3

Zu 22 ml N,N-Dimethylanilin tropft man bei Raumteperatur 50 ml Phosphoroxychlorid ($POCl_3$), dann werden unter Kühlung, so dass die Temperatur unterhalb 40°C bleibt, 22,3 g 2-para-Chlorphenyl-4,6-dihydroxy-pyrimidin portionenweise sugegeben und 2 Stunden bei Raumtemperatur gerührt, nachher noch 2 Stunden unter Rückfluss gekocht. Das Reaktionsgemisch wird anschliessend am Rotationsverdampfer ein geengt und der Rückstand mit 500 ml Wasser zerrieben. Das Produkt wird abgenutscht, in Methylenchlorid gelöst, mit Bleicherde behandelt, getrocknet und eingeengt. Der Rückstand kristallisiert und ergibt 16,2 g 2-para-Chlorphenyl-4,6-dichlorpyrimidin mit Schmelzpunkt 119—120°C. Eine bei 80°/0,02 mbar sublimierte Probe schmilzt bei 120—121°C.

## Beispiel 5

2-para Methoxyphenyl-4,6-dihydroxy-pyrimidin

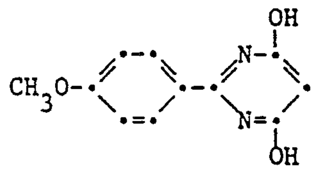

Zwischen produkt fur Verbindung No. 6

Zu einer Suspension von 112 g para-Methoxybenzamid-Hydrochlorid und 101 g Malonsäurediäthylester in 520 ml Aethanol werden innerhalb von 10 Minuten 338 g 30%ige Natriummethylat/Methanol-Lösung gegeben und das Ganze anschliessend während 5 Stunden am Rückfluss gekocht. Das Reaktionsgemisch wird anschliessend am Rotationsverdampfer eingeengt und der Rückstand in 1000 ml 80°C warmem Wasser gelöst. Man filtriert und säuert das Filtrat bis pH 1 an. Der Niederschlag wird abfiltriert und im Vakuum bei 80°C getrocknet. Man erhält so 109,8 g 2-para Methoxyphenyl-4,6-dihydroxypyrimidin mit einem Schmelzpunkt von 319°C (Zersetzung).

## Beispiel 6

2-para Methoxyphenyl-4,6-dichlor-pyrimidin

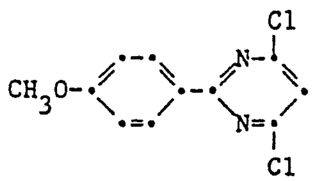

Verbindung No. 6

Zu 54,5 g 2-para-Methoxyphenyl-4,6-dihydroxy-pyrimidin tropft man unter Kühlen, so dass die Reaktionstemperatur 45°C nicht überschreitet, innerhalb 30 Minuten 126 ml Phosphoroxychlorid ($POCl_3$) und anschliessend 57 ml N,N-Dimethylanilin. Das Reaktionsgemisch wird anschliessend während 2 Stunden bei Raumtemperatur gerührt und dann noch 2 Stunden unter Rückfluss gekocht. Dann wird am Rotationsverdampfer eingeengt und der Rückstand in 2 l Eis/Wasser zerrieben. Das feste Produkt wird abgenutscht, in 1,5 l Methylenchlorid gelöst, mit Bleicherde behandelt und getrocknet. Nach dem Filtrieren und Einengen der Methylenchlorid-Lösung erhält man einen Rückstand, der sich aus Aethanol umkristallisieren lässt. Man erhält so 51 g kristallines 2-para-Methoxyphenyl-4,6-dichlor-pyrimidin vom Schmelzpunkt 127—128°C.

In analoger Weise zu diesen Beispielen werden folgende Verbindungen hergestellt:

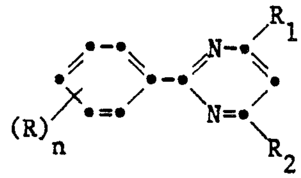

| No. | $(R)_n$ | $R_1$ | $R_2$ | phys. Daten |
|---|---|---|---|---|
| 1 | $(H)_5$ | Cl | Cl | Smp. 95—96° |
| 2 | 4-$CH_3$ | Cl | Cl | Smp. 86—87° |
| 3 | 4-$CH_3$ | Br | Br | Smp. 125—126° |
| 4 | $(H)_5$ | Br | Br | Smp. 115—118° |
| 5 | 4-Cl | Cl | Cl | Smp. 120° |
| 6 | 4-$OCH_3$ | Cl | Cl | Smp. 127—128° |
| 7 | 4-CN | Cl | Cl | Smp. 230—232° |
| 8 | 3-$CF_3$ | Cl | Cl | Smp. 56—57° |
| 9 | 2-$CH_3$ | Cl | Cl | Smp. 74—75° |
| 10 | 3-Cl, 4-F | Cl | Cl | Smp. 94—95° |
| 11 | 2,6$(CH_3)_2$ | Cl | Cl | Smp. 103—104° |
| 12 | 3,4$(CH_3)_2$ | Br | Br | |
| 13 | 4-$C_3H_7$i | Cl | Cl | Smp. 63—64° |
| 14 | 4-$C_3H_7$i | Cl | F | |
| 15 | 4-Cl | F | F | Smp. 139—141° |
| 16 | 2-$CH_3$, 6-$C_2H_5$ | Cl | Cl | |
| 17 | 2-Cl | Cl | Cl | Smp. 116—118° |
| 18 | 3-$C_2H_5$ | Cl | Cl | |
| 19 | 3-$CHF_2$ | Cl | Cl | |
| 20 | 2,4$(CH_3)_2$ | Br | Br | |
| 21 | 2,3,6$(CH_3)_2$ | Cl | Cl | |
| 22 | 3-$C_3H_7$i | Cl | Cl | |
| 23 | 4-$CHF_2$ | Cl | Cl | |
| 24 | 2-Cl, 4-$CH_3$ | Br | Br | |
| 25 | 4-$OCH_2CH{=}CH_2$ | Cl | Cl | Smp. 57—58° |
| 26 | 4-OH | F | F | |
| 27 | 4-$COCH_3$ | Cl | Cl | Smp. 129—130° |

| No. | (R)$_n$ | $R_1$ | $R_2$ | phys. Daten |
|---|---|---|---|---|
| 28 | 4-$OCOC_2H_5$ | Cl | Cl | |
| 29 | 4-$OCH(CH_3)COOCH_3$ | Cl | Cl | |
| 30 | 4-OH | Cl | Cl | Smp. 135—137° Mp. |
| 31 | 4-$OCON(CH_3)_2$ | Cl | Cl | Smp. 191—193° |
| 32 | 3-$CH_2F$ | Cl | Cl | |
| 33 | 4-$CH_2F$ | Cl | Cl | |
| 34 | 4-$COOCH_3$ | Cl | Cl | Smp. 135—140° |
| 35 | 4-Cl, 2,6-$(OCH_3)_2$ | Cl | Cl | |
| 36 | 4-CHO | Cl | Cl | Smp. 160—162° |
| 37 | 3-$CH_2Cl$ | Cl | Cl | |
| 38 | 3,5$(CF_3)_2$ | Cl | Cl | |
| 39 | 4-$CF_3$ | Br | Br | |
| 40 | 4-$OCHF_2$ | Cl | Cl | |
| 41 | 3,5$(OC_2H_5)_2$ | Cl | Cl | |
| 42 | 4-$OC_3H_7$i | F | F | |
| 43 | 3-$NO_2$ | Cl | Cl | Smp. 136—138° |
| 44 | 4-$NO_2$ | Cl | Cl | Smp. 167—168° |
| 45 | 3-$NO_2$, 4-$CH_3$ | Cl | Cl | |
| 46 | 2-Cl, 4-$NO_2$ | Cl | Cl | |
| 47 | 2-$N(CH_3)_2$ | Cl | Cl | |
| 48 | 3-$NHCOCH_3$ | Br | Br | |
| 49 | 3-$NHCOCH_2Cl$ | Cl | Cl | |
| 50 | 3-$OCF_2Cl$, 5-Cl | Cl | Cl | |
| 51 | 2-$CON(CH_3)_2$ | Cl | Cl | |
| 52 | 4-$OCF_2CHF_2$ | Cl | Cl | |
| 53 | 4-$CONHC_4H_9$n | Cl | Cl | |
| 54 | 4-$NHCOCH_2Cl$ | Cl | Cl | Smp. 196—198° |
| 55 | 4-$COC_3H_7$n | Cl | Cl | |
| 56 | 4-$OCF_2CHFCl$ | Cl | Cl | |
| 57 | 2-OH | Cl | Cl | |
| 58 | 4-$COOCH_2CH{=}CH_2$ | Cl | Cl | |

| No. | $(R)_n$ | $R_1$ | $R_2$ | phys. Daten |
|---|---|---|---|---|
| 59 | 4-$COOCH_2C\equiv CH$ | Cl | Cl | Smp. 105—109° |
| 60 | 2-Cl, 6-$C\equiv CH$ | Cl | Cl | |
| 61 | 3-$C\equiv C—C(CH_3)_2OCH_3$ | Br | Br | |
| 62 | 4-$C\equiv C—C(CH_3)_2OH$ | Cl | Cl | |
| 63 | 4-$C\equiv C—C(CH_3)_2OCH_3$ | Cl | Cl | |
| 64 | 3,5$(I)_2$, 4-$OCH(CH_3)COOCH_3$ | Cl | Cl | |
| 65 | 4-$CH=CH—C_4H_9n$ | Cl | Cl | |
| 66 | 4-OH | Br | Br | |
| 67 | 4-Br | Cl | Cl | Smp. 130—131° |
| 68 | 3-OH | Cl | Cl | Smp. 144—146° |
| 69 | 3-$OCH_3$ | Cl | Cl | Smp. 97—100° |
| 70 | 3-$OCOCH_2Cl$ | Cl | Cl | |
| 71 | 2-$OCH_3$ | Cl | Cl | Smp. 67—70° |
| 72 | 2,6$(F)_2$ | Cl | Cl | |
| 73 | 4-F | Cl | Cl | Smp. 102—105° |
| 74 | 3-Cl, 4-$CH_3$ | Cl | Cl | Smp. 91—92° |
| 75 | $(H)_5$ | F | F | Smp. 114—116° |
| 76 | $(H)_5$ | F | Cl | Smp. 105° |
| 77 | 2,5$(Cl)_2$, 4-OH | F | F | |
| 78 | 2-Cl, 4-$OCH(CH_3)COOC_2H_5$ | Cl | Cl | |
| 79 | 3-$SO_2N(CH_3)_2$ | Cl | Cl | |
| 80 | 4-$CSN(CH_3)_2$ | Cl | Cl | |
| 81 | 4-$C(CH_3)=CH_2$ | Cl | Cl | |
| 82 | 4-$CH_2COOCH_3$ | Cl | Cl | |
| 83 | 4-$CH_2PO(OC_2H_5)_2$ | Cl | Cl | Smp. 110—112° |
| 84 | 4-$CH_2CH=CH_2$ | Cl | Cl | |
| 85 | 3-$C\equiv CH$, 5-$CH_3$ | Cl | Cl | |
| 86 | 2-$C\equiv CH$ | Cl | Cl | |
| 87 | 4-$C\equiv CH$ | Cl | Cl | Smp. 168—170° |
| 88 | 2-$CH_3$, 5-$N(CH_3)_2$ | Cl | Cl | |
| 89 | 2-$CH_3$, 5-Cl | Cl | Cl | |

| No. | $(R)_n$ | $R_1$ | $R_2$ | phys. Daten |
|---|---|---|---|---|
| 90 | 3-Br, $4-OC_3H_7n$ | Cl | Cl | |
| 91 | $3-NO_24-Cl$ | Cl | Cl | Smp. 158—159° |
| 92 | $3-NH_2$, 4-Cl | Cl | Cl | |
| 93 | $3-CH_3$, $4-NO_2$ | Cl | Cl | Smp. 173—175° |
| 94 | $3-CH_3$, $4-NH_2$ | Cl | Cl | fest |
| 95 | 2-Cl, $5-CF_3$ | Cl | Cl | |
| 96 | $3-CF_3$, 4-Cl | Cl | Cl | |
| 97 | $2,6(OCH_3)_2$, $3-NO_2$ | Cl | Cl | |
| 98 | $2,6(OCH_3)_2$ $3-NH_2$ | Cl | Cl | |
| 99 | $2,6(OCH_3)_2$, $3-NHCOCH_3$ | Cl | Cl | |
| 100 | $2-CH_3$, $6-C_2H_5$, $4-OCON(CH_3)_2$ | Cl | Cl | |
| 101 | $3,5(I)_2$, 4-OH | Cl | Cl | |
| 102 | $3,5(I)_2$, $4-OCH_3$ | Br | Br | |
| 103 | $3,5(Br)_2$, 4-OH | Cl | Cl | |
| 104 | $3,5(Br)_2$, $4-OCH_2-CH=CH_2$ | Cl | Cl | |
| 105 | $3,4,5(OCH_3)_2$ | Cl | Cl | Smp. 167—169° |
| 106 | $2,3(Cl)_2$ | Cl | Cl | Smp. 116—118° |
| 107 | 4– 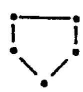 | Cl | Cl | |
| 108 | 3– 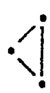 | Br | Br | |
| 109 | 3– 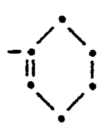 | Cl | Cl | |
| 110 | $3-NH_2$ | Cl | Cl | fest |
| 111 | $4-NH_2$ | Cl | Cl | fest |
| 112 | $3-NHCOCH_3$ | Cl | Cl | Smp. 228—230° |
| 113 | $4-NHCOCH_3$ | Cl | Cl | Smp. 190—192° |
| 114 | $4-CH_3$, $3-SO_2H$ | Cl | Cl | |
| 115 | $4-CH_3$, $3-SO_2NH_2$ | Cl | Cl | |
| 116 | $4-NHCONHC_2H_5$ | Cl | Cl | |
| 117 | 4-Cl, $3-NH_2$ | Cl | Br | |

| No. | $(R)_n$ | $R_1$ | $R_2$ | phys. Daten |
|---|---|---|---|---|
| 118 | 3-I | Cl | Cl | |
| 119 | 3-COOH | Cl | Cl | Smp. 250° |
| 120 | 4-COOH | Cl | Cl | Smp. 236—238° |
| 121 | 4-$N(CH_3)_2$ | Cl | Cl | Smp. 150—155° |
| 122 | 3$NHCH_3$ | Cl | Cl | |
| 123 | 3-NHCO 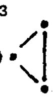 | Cl | Cl | |
| 124 | 4-$OCH_2OCH_3$ | Cl | Cl | |
| 125 | 4-$SCH_3$ | Cl | F | |
| 126 | 3-SH | Cl | Cl | |
| 127 | 4-$SCH_3$ | Cl | Cl | Smp. 109—111° |
| 128 | 4-$OCOOCH_3$ | Cl | F | |
| 129 | 3-$OCOOCH_3$ | Cl | Cl | |
| 130 | 3-F | Cl | Cl | Smp. 72—74° |
| 131 | 4-$OC_2H_5OC_2H_5$ | Cl | Cl | Smp. 75—77° |
| 132 | 4-$OC_2H_4OC_3H_7n$ | Cl | Cl | Wachs |
| 133 | 4-$SO_2CH_3$ | Cl | Cl | Smp. 163—165° |
| 134 | 4-$OC_2H_4OC_2H_4OC_2H_5$ | Cl | Cl | Smp. 42—43° |
| 135 | 4-$OCH_3$ | Br | Br | Smp. 129—131° |
| 136 | —$OC_6H_{13n}$ | Cl | Cl | |
| 137 | 4-$OCH_2$—C=CH | Cl | Cl | |
| 138 | 4-$OC_2H_4(C_2H_5)_2$ | Cl | Cl | |
| 139 | 4-$OC_2H_4Cl$ | Cl | Cl | |
| 140 | 4-$OC_2H_4OH$ | Cl | Cl | |
| 141 | 4-$OC_2H_4SCH_3$ | Cl | Cl | |
| 142 | 4-$OC_2H_4C_2H_4Cl$ | Cl | Cl | Smp. 88—89° |
| 143 | 4-$OCF_3$ | Cl | Cl | |
| 144 | 5-$OC_2H_5$ | Cl | Cl | |
| 145 | 4-$OCOCH_3$ | Cl | Cl | Smp. 113—115° |
| 146 | 4-$OCH(CH_3)COOCH_3$ | Cl | Cl | |
| 147 | 4-$OCH(CH_3)COOCH_3$ | Br | Cl | Smp. 118—120° |
| 148 | 4-$OCOCH=CH_2$ | Cl | Cl | |

| No. | (R)$_n$ | $R_1$ | $R_2$ | phys. Daten |
|---|---|---|---|---|
| 149 | 4-$OCOC_3H_6CH=CH_2$ | Cl | Cl | |
| 150 | 4-$OCH_2CON(CH_3)_2$ | Cl | Cl | |
| 151 | 4-$OCH_2CH=CHCH_3$ | Cl | Cl | |
| 152 | 4-$OC_2H_4CH=CClCH_3$ | Cl | Cl | |
| 153 | $(H)_5$ | Cl | Br | |
| 154 | $(H)_5$ | J | J | |
| 155 | 4-$SOCH_3$ | Cl | Cl | |
| 156 | 4-$SC_2H_4N(CH_3)_2$ | Cl | Cl | |
| 157 | 4-$SC_2H_4OCH_3$ | Cl | Cl | |
| 158 | 4-$SC_6H_{13n}$ | Cl | Cl | |
| 159 | 4-$SC_2H_4COOC_4H_9$ | Cl | Cl | |
| 160 | 4-$SCOCH_3$ | Cl | Cl | |
| 161 | 4-$SCH_2CH=CH_2$ | Cl | Cl | |
| 162 | 4-$NH_2$ | Br | Br | |
| 163 | 4-$NHC_6H_{13n}$ | Cl | Cl | |
| 164 | 4-$NHC_3H_{7i}$ | Cl | Cl | |
| 165 | 4-$NHCH_2COOCH_3$ | Cl | Br | |
| 166 | 4-$NHCH(CH_3)CON(CH_3)_2$ | Cl | Cl | |
| 167 | 4-$NHCOCH=CH_2$ | Cl | Cl | |
| 168 | 4-$NHCH_2—CH=CH_2$ | Cl | Cl | |
| 169 | 4-$N(CH_2—CH=CH_2)_2$ | Cl | Cl | |
| 170 | 4-$NHCH—C≡CH$ | Cl | Cl | |
| 171 | 4-$NHCH_2CH=CHC_2H_5$ | Cl | Cl | |
| 172 | 4-$NH(CH_2)_4C≡CH$ | Cl | Cl | |
| 173 | 4-$NHOCH_3$ | Cl | Cl | |
| 174 | 4-$NHOCH_2CH=CH_2$ | Cl | Cl | |
| 175 | 4-$N(CH_3)OCH_3$ | Cl | Cl | |
| 176 | 4-$N(CH_3)COCH_3$ | Cl | Cl | |
| 177 | 4-NH 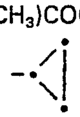 | Cl | Cl | |
| 178 | 4-NH 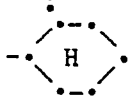 | Cl | Cl | |
| 179 | 4-$NHCOOCH_3$ | Cl | Cl | |

| No. | $(R)_n$ | $R_1$ | $R_2$ | phys. Daten |
|---|---|---|---|---|
| 180 | 4-$N(CH_3)COOC_3H_{7i}$ | Cl | Cl | |
| 181 | 4-$NHCONHCH_3$ | Cl | Cl | |
| 182 | 4-N-$NHCON(CH_3)_2$ | Cl | Cl | |
| 183 | 4-$N(CH_3)CONHCH_3$ | Cl | Cl | |
| 184 | 4-$N(CH_3)CON(CH_3)OCH_3$ | Cl | Cl | |
| 185 | 4-$OCOCH_2CH{=}CHCH_3$ | Cl | Cl | |
| 186 | 4-$CONH_2$ | Cl | Cl | |
| 187 | 4-$CON(C_3H_7n)_2$ | Cl | Cl | |
| 188 | 4-$CONHC_6H_{13}n$ | Cl | Cl | |
| 189 | 4-$OCOC_2H_4N(C_2H_5)_2$ | Br | Br | |
| 190 | 4-$CONHOCH_3$ | Cl | Cl | |
| 191 | 4-$CON(CH_3)_2$ | Cl | Cl | |
| 192 | 4-CHO | Br | Br | |
| 193 | 4-$COC_4H_9n$ | Cl | Cl | |
| 194 | 4-$COCH{=}CH{-}N(CH_3)_2$ | Cl | Cl | |
| 195 | 4-$CSN(C_3H_7)_2$ | Cl | Cl | |
| 196 | 4-$CSNHC_6H_{13}n$ | Cl | Cl | |
| 197 | 4-$N{=}CHC_3H_7i$ | Cl | Cl | |
| 198 | 4-$N{=}CHC_6H_{13}n$ | Cl | Cl | |
| 199 | 4-$N{=}CH(CH_3)_2$ | Cl | Cl | |
| 200 | 4-$N(CH_3)CH_2OCH_3$ | Cl | Cl | |
| 201 | 4-$SO_2NH_2$ | Cl | Cl | |
| 202 | 4-$SO_2N(CH_3)_2$ | Cl | Cl | |
| 203 | 4-$SO_2NHC_4H_9$ | Cl | Cl | |
| 204 | 4-$SO_2NHCH_2CH{=}CH_2$ | Cl | Cl | |
| 205 | 4-$CH(OCH_3)_2$ | Cl | Cl | |
| 206 | 4-$CH(OC_2H_4OCH_3)_2$ | Cl | F | |
| 207 | 4-$(C_4H_9n)(OC_2H_5)_2$ | Cl | Cl | |
| 208 | 4-$C(CH_3)(OCH_3)_2$ | Cl | Cl | |
| 209 | 4-$C(CH_3)(OC_2H_4SCH_3)_2$ | Cl | Cl | |
| 210 | 4-$SO_3H$ | Cl | Cl | |

| No. | $(R)_n$ | $R_1$ | $R_2$ | phys. Daten |
|---|---|---|---|---|
| 211 | $4\text{-}CF_3$ | Cl | Cl | |
| 212 | $4\text{-}CH_2Br$ | Cl | Cl | Smp. 155—156° |
| 213 | $4\text{-}CH_2Cl$ | Cl | Cl | |
| 214 | $4\text{-}CH_2OCH_3$ | Cl | Cl | |
| 215 | $4\text{-}CH_2OH$ | Cl | Cl | |
| 216 | $4\text{-}CH_2OCOCH_3$ | Cl | Cl | Smp. 108—110° |
| 217 | $4\text{-}CH_2OC_4H_9n$ | Cl | Cl | |
| 218 | $4\text{-}CH_2SCH_3$ | Cl | Cl | |
| 219 | $4\text{-}CH_2N(CH_3)_2$ | Cl | Cl | |
| 220 | $4\text{-}CHClCH_3$ | Cl | F | |
| 221 | $4\text{-}C_2H_5$ | Cl | Cl | |
| 222 | $4\text{-}C_6H_{13}n$ | Cl | Cl | |
| 223 | $4\text{-}C_5H_{11}iso$ | Br | Br | |
| 224 | $4\text{-}CH{=}CH_2$ | Cl | Cl | |
| 225 | $4\text{-}CH_2{—}CH{=}CH_2$ | Cl | F | |
| 226 | $4\text{-}CCl{=}CH_2$ | Cl | Cl | Smp. 128—130° |
| 227 | $4\text{-}C_2H_4Cl$ | Cl | Cl | |
| 228 | $4\text{-}C_2H_4N(C_2H_5)_2$ | Cl | Cl | |
| 229 | 4- 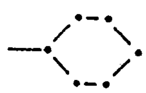 | Cl | Cl | |
| 230 | 4 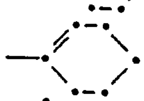 | Cl | Cl | |
| 231 | 4- 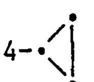 | Cl | Cl | |
| 232 | $4\text{-}CH{=}CH_2{—}CH_2OCH_3$ | Cl | Cl | |
| 233 | $4\text{-}N(CH_3)COCH_2CL$ | Cl | Cl | |
| 234 | $4\text{-}CH_2CN$ | Cl | Cl | Smp. 151—158° |
| 235 | 3-F | Br | Br | |
| 236 | 3-Cl | Cl | Cl | Smp. 117—119° |
| 237 | $3\text{-}NO_2$ | Br | Br | Smp. 165—167° |
| 238 | $3\text{-}NO_2$ | F | F | |
| 239 | $3\text{-}OC_3H_7i$ | Cl | Cl | |
| 240 | $3\text{-}OCH_2CH{=}CHCH_3$ | Cl | F | |
| 241 | $3\text{-}OCH_2C{=}CH$ | Cl | Cl | |

| No. | (R)n | $R_1$ | $R_2$ | phys. Daten |
|---|---|---|---|---|
| 242 | 3-$OC_2H_4N(C_2H_5)_2$ | Cl | Cl | |
| 243 | 3-$OCH(CH_3)CH_2N(C_2H_5)$ | Cl | Cl | |
| 244 | 3-$OC_2H_4Cl$ | Cl | Cl | |
| 245 | 4-$CH_3$ | Br | Cl | |
| 246 | 3-$OC_2H_4SC_2H_5$ | Cl | Cl | |
| 247 | 3-$OC_2H_4OC_3H_7$n | Cl | Cl | |
| 248 | 3-$OCF_3$ | Cl | F | |
| 249 | 3-$OCHF_2$ | Cl | Cl | |
| 250 | 3-$OCF_2CHF_2$ | Cl | Cl | |
| 251 | 3-$OCF_2CHFCl$ | Br | Br | |
| 252 | 3-$OCOC_2H_5$ | Cl | Cl | |
| 253 | 3-$OCOCH_2Cl$ | Br | Br | |
| 254 | 3-$SC_2H_5$ | Cl | Cl | |
| 255 | 3-$SCF_3$ | Cl | Cl | |
| 256 | 3-$SCHF_2$ | Cl | Cl | |
| 257 | 3-$SO_2CH_3$ | Cl | Cl | |
| 258 | 3-$SC_3H_6N(CH_3)_2$ | Cl | Cl | |
| 259 | 3-$SC_3H_6Cl$ | Cl | Cl | |
| 260 | 3-$SC_5H_{11}$iso | Cl | Cl | |
| 261 | 3-$SCH_2COOC_3H_7$n | Cl | F | |
| 262 | 3-$SCH_2C{=}CH$ | Cl | Cl | |
| 263 | 3-$NH_2$ | Br | Br | fest |
| 264 | 3-$N(CH_3)_2$ | Cl | Cl | |
| 265 | 3-$NHC_4H_9$n | Cl | Cl | |
| 266 | 3-$NHC_4H_9$sek | Cl | Cl | |
| 267 | 3-$N(CH_2C{=}CH)_2$ | Cl | F | |
| 268 | 3-$NHCH_2CH{=}CH_2$ | Cl | Cl | |
| 269 | 3-$N(CH_3)COCH_2Cl$ | Cl | Cl | |
| 270 | 3-$NHCOCH{=}CH{-}CH_3$ | Cl | Cl | |
| 271 | 3-NHOH | Br | Br | |
| 272 | 3-$NHOC_2H_5$ | Cl | Br | |

| No. | $(R)_n$ | $R_1$ | $R_2$ | phys. Daten |
|---|---|---|---|---|
| 273 | 3-$NCH_3OCH_3$ | Cl | Cl | |
| 274 | 3-$N(C_3H_7iso)CO_2C_2H_5$ | Cl | Cl | |
| 275 | 3-$NHCOOC_4H_9iso$ | Cl | Cl | |
| 276 | 3-$NHCONHC_4H_9n$ | Cl | Cl | |
| 277 | 3-$N(CH_3)CON(CH_3)_2$ | Cl | Cl | |
| 278 | 3-COOH | Cl | Cl | Smp. 250° |
| 279 | 3-$COOCH_3$ | Cl | Cl | Smp. 190—191° |
| 280 | 3-$COOCH_2CH{=}CH_2$ | Cl | Cl | Smp. 120—121° |
| 281 | 3-$COOC_3H_7i$ | Cl | Cl | |
| 282 | 3-$COCH_2—C{\equiv}C—C_3H_7n$ | Cl | Cl | |
| 283 | 3-$CONH_2$ | Cl | Cl | |
| 284 | 3-$CON(CH_3)_2$ | Cl | Cl | |
| 285 | 3-$CONHCH_3$ | Br | Br | |
| 286 | 3-$CONHCH_2CH{=}CH_2$ | Cl | F | |
| 287 | 3-$COOC_2H_6N(CH_3)_2$ | Cl | Cl | |
| 288 | 3-CHO | Cl | Cl | |
| 289 | 3-$COCH_3$ | Cl | Cl | |
| 290 | 3-CO- 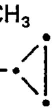 | Cl | Cl | |
| 291 | 3-$CSNHC_4H_9n$ | Cl | Cl | |
| 292 | 3-$CSNHC_3H_7iso$ | Cl | Cl | |
| 293 | 3-$N{=}CHC_3H_7(i)$ | Cl | Cl | |
| 294 | 3-$N{=}C(CH_3)CH_2OCH_3$ | Cl | Cl | |
| 295 | 3-$N{=}CHC_2H_4OC_2H_5$ | Cl | Cl | |
| 296 | 3-$SO_2NH_2$ | Cl | Cl | Smp. 207—208° |
| 297 | 3-$SO_2NHCH_3$ | Cl | Cl | Smp. 174—175° |
| 298 | 3-$SO_2N(C_4H_9n)_2$ | Cl | Cl | |
| 299 | 3-$SO_2N(CH_3)_2$ | Cl | Cl | Smp. 144—145° |
| 300 | 3-$SO_3H$ | Cl | Cl | Smp. 95—96° |
| 301 | 3-$CF_3$ | Cl | F | |
| 302 | 3-$CH_2CN$ | Cl | Cl | |
| 303 | 3-$CH_2Cl$ | Cl | Cl | |

| No. | $(R)_n$ | $R_1$ | $R_2$ | phys. Daten |
|---|---|---|---|---|
| 304 | $3\text{-}CH_2OC_2H_5$ | Cl | Cl | |
| 305 | $3\text{-}CH_2OH$ | Br | Br | |
| 306 | $3\text{-}C_2H_4SCH_3$ | Cl | Cl | |
| 307 | $3\text{-}C_2H_4SOCH_3$ | Cl | Cl | |
| 308 | $3\text{-}CHCl{-}C_2H_5$ | Cl | F | |
| 309 | $3\text{-}C_3H_7n$ | Cl | Cl | |
| 310 | $3\text{-}C_6H_{13}iso$ | Cl | Cl | |
| 311 | $3\text{-}C{\equiv}CH$ | Cl | Cl | |
| 312 | $3\text{-}C{\equiv}CCH_3$ | Cl | Cl | |
| 313 | $3\text{-}CH{=}CH_2$ | Cl | Cl | |
| 314 | $3\text{-}CCl{=}CH_2$ | Cl | Cl | |
| 315 | $3\text{-}CCl{=}CHCH_3$ | Cl | Cl | |
| 316 | $3\text{-}C_2H_4N(CH_3)_2$ | Cl | Cl | |
| 317 | 3- 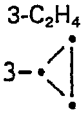 | Cl | Cl | |
| 318 | $3\text{-}CH{=}CH{-}C_3H_7n$ | Cl | Cl | |
| 319 | 3- 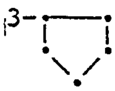 | Cl | Cl | |
| 320 | $3\text{-}CH_2COOC_2H_5$ | Cl | Cl | |
| 321 | $3\text{-}CH_2CONH_2$ | Br | Br | |
| 322 | $2\text{-}CH_3$ | Br | Br | |
| 323 | 2-F | Cl | Cl | |
| 324 | $2\text{-}OCH_2C{=}CH$ | Cl | Cl | |
| 325 | $2\text{-}SCH_3$ | Cl | Cl | |
| 326 | 2-SH | Cl | Cl | |
| 327 | 2-COOH | Cl | Cl | |
| 328 | $2\text{-}COOCH_3$ | Cl | Cl | |
| 329 | $2\text{-}CH_2OH$ | Br | Br | |
| 330 | 2-CHO | Cl | Cl | |
| 331 | 3-Cl, 4F | F | F | Smp. 101—103° |
| 332 | 3-Cl, 4F | F | Cl | |
| 333 | $3,4(CH_3)_2$ | Cl | Cl | |
| 334 | $3,5(Cl)_2$ | Cl | Cl | Smp. 175—177° |

| No. | $(R)_n$ | $R_1$ | $R_2$ | phys. Daten |
|---|---|---|---|---|
| 335 | $2,6(Cl)_2$ | Cl | Cl | |
| 336 | $2,3(CH_3)_2$ | Cl | Cl | |
| 337 | $2,4(CH_3)_2$ | Cl | Cl | |
| 338 | 3-Cl, 4-$C_3H_7$iso | Cl | Cl | |
| 339 | 2-Cl, 4-$CH_3$ | Cl | Cl | |
| 340 | $3,4(Cl)_2$ | Cl | Cl | |
| 341 | $3,5(OCH_3)_2$ | Cl | Cl | Smp. 168—172° |
| 342 | 3-$NH_2$, 4-$CH_3$ | Cl | Cl | |
| 343 | 3-$NHCH_3$, 4-$CH_3$ | Cl | Cl | |
| 344 | 3-OH, 5-Cl | Cl | Cl | |
| 345 | 3-$OCHF_2$, 5-Cl | Cl | Cl | |
| 346 | 3-$OCH_3$, 5-Cl | Cl | Cl | |
| 347 | 3-COOH, 5-Cl | Cl | Cl | |
| 348 | 3-$COOCH_3$, 5-Cl | Cl | Cl | |
| 349 | 3-$CONH_2$, 5-Cl | Cl | Cl | |
| 350 | 4-$COCH_3$, 4-$CH_3$ | Cl | Cl | |
| 351 | 2-Cl, 6-C=CH | Br | Br | |
| 352 | 4-$SO_2N(CH_3)_2$, 5-$CH_3$ | Br | Br | |
| 353 | 3-C≡CH, $5CH_3$ | Cl | F | |
| 354 | 2-$CH_3$, 5-$N(CH_3)_2$ | Cl | Cl | |
| 355 | 2-$CH_3$, 5-Cl | Cl | Cl | |
| 356 | 3-Br, 4-$OCH_3$ | Cl | Cl | |
| 357 | 3-$CH_3$, 4-$NHCON(CH_3)_2$ | Cl | Cl | |
| 358 | 2 $SCH_3$, 5-$NO_2$ | Cl | Cl | |
| 359 | 2 $SCH_3$, 5-$NH_2$ | Cl | Cl | |
| 360 | 2 Cl, 5-$NO_2$ | Cl | Cl | |
| 361 | 2 Cl, $5NH_2$ | Cl | Cl | |
| 362 | 3,4 $(OCH_3)_2$ | Cl | Cl | |
| 363 | 3,4 $(OH)_2$ | Cl | Cl | |
| 364 | 2,3 $(Cl)_2$ | Cl | Cl | Smp. 116—118° |
| 365 | 2,5 $(OCH_3)_2$ | Cl | Cl | Smp. 127—129° |

| No. | $(R)_n$ | $R_1$ | $R_2$ | phys. Daten |
|---|---|---|---|---|
| 366 | 2,5 $(OH)_2$ | Cl | Br | |
| 367 | 4-OH, 3-$CH_3$ | Cl | Br | |
| 368 | 4-$OCH_3$, 3-$NO_2$ | Cl | Cl | |
| 369 | 4-OH, 3-$NO_2$ | Cl | Cl | |
| 370 | 4-$OCH_3$, 3-$NH_2$ | Cl | Cl | |
| 371 | 4-OH, 3-$NH_2$ | Cl | Cl | |
| 372 | 3,5 $(OH)_2$ | Cl | Cl | |
| 373 | 2,6 $Cl_2$, 3-$NO_2$ | Cl | Cl | |
| 374 | 2,6 $Cl_2$, 3-$NH_2$ | Cl | Cl | |
| 375 | 2,6$(OCH_3)_2$, 4-Cl | Br | Br | |
| 376 | 2,6$(OH)_2$, 4-Cl | Br | Br | |
| 377 | 3,5$(J)_2$, 4-$OCH_3$ | Cl | Cl | |
| 378 | 3,5$(J)_2$, 4-OH | Br | Br | |
| 379 | 3,5$(Cl)_2$, 4-$OCH_3$ | Br | Br | |
| 380 | 3,5$(Cl)_2$, 4-OH | Cl | Cl | |
| 381 | 2,5$(Cl_2)$, 4-$OCH_3$ | Cl | Cl | |
| 382 | 2,5$(Cl)_2$, 4-OH | Cl | Cl | |
| 383 | 4F | Cl | Br | |
| 384 | 3,4$(OH)_2$ | Br | Br | |
| 385 | 2,6$(OH)_2$, 3-$NH_2$ | Cl | Cl | |
| 386 | 3-$OCH_2OCH_3$ | Cl | Cl | |
| 387 | 3,5$(OH)_2$, 4-$OCH_3$ | Cl | Cl | |
| 388 | 3,4,5$(OH)_3$ | Cl | Cl | |
| 389 | 2,3,4$(OCH_3)_3$ | Cl | Cl | |
| 390 | 2,3,4$(OH)_3$ | Cl | Cl | |
| 391 | 3-$SCH_2CO_2CH_3$ | Cl | Cl | |
| 392 | 4-OH, 3$CH_3$ | Cl | Cl | |
| 393 | 3-$CH_3$ | Cl | Cl | Smp. 76—79° |
| 394 | 3-$SCH_3$ | Cl | Cl | Smp. 103—105° |
| 395 | 3-$CCl_3$ | Cl | Cl | |
| 396 | 4-$CCl_3$ | Cl | Cl | |

| No. | $(R)_n$ | $R_1$ | $R_2$ | phys. Daten |
|---|---|---|---|---|
| 397 | 4-OH, 3-$CH_3$ | Br | Cl | Smp. 140—145° |
| 398 | 4-$SCH_2COCH_3$ | Cl | Cl | |
| 399 | 3-$SCOCH=CH_2$ | Cl | Cl | |
| 400 | 4-$SCOCH=CHCH_3$ | Cl | Cl | |
| 401 | 3-SCO—$CH_2{\equiv}CH$ | Br | Br | |
| 402 | 4-$SOCH_2CH{\equiv}CH_2$ | Cl | Cl | |
| 403 | 3-$SOCH_2CH=CH_2$ | Cl | Cl | |
| 404 | 4-$SOCH_2C{\equiv}CH$ | F | Cl | |
| 405 | 3-$SOCH_2C{\equiv}CH$ | Cl | Cl | |
| 406 | 3-$SO_2CH_2CH=CH_2$ | Br | Br | |
| 407 | 4-$SO_2CH_2CH=CH_2$ | Cl | Cl | |
| 408 | 3-$SO_2CH_2C{\equiv}CH$ | Cl | Cl | |
| 409 | 4-$SO_2CH_2C{\equiv}CH$ | Cl | Cl | |
| 410 | 4-$OCOC_6H_{13n}$ | Cl | Cl | |
| 411 | 3-OCO—$C_5H_{11}$i | Cl | Cl | |
| 412 | 4-$OCONHCH_3$ | Cl | Cl | Smp. 205—209° |
| 413 | 3-$OCONHCH_3$ | Cl | Cl | Smp. 134—137° |
| 414 | 4-$OCON(CH_3)_2$ | Cl | Cl | Smp. 191—193° |
| 415 | 4-$OCONHC_4H_9$ | Cl | Cl | |
| 416 | 3-$OCONHC_3H_7$i | Cl | Cl | |
| 417 | 4-$OCOCH=CH_2$ | Cl | Cl | |
| 418 | 3-$OCOCH=CH$—$CH_3$ | Cl | Cl | |
| 419 | 4-$OCOCH_2OCH_3$ | Cl | Cl | |
| 420 | 3-$OCON(CH_3)_2$ | Cl | Cl | |
| 421 | 4-$NHCONHC_4H_9$n | Cl | Cl | |
| 422 | 4-SH | Cl | Cl | |
| 423 | 3-$OC_2H_4OH$ | Cl | Cl | |
| 424 | 3-$NHC_2H_4COOCH_3$ | Cl | Cl | |
| 425 | 3-Br, 4-OH | Cl | Cl | |
| 426 | 3-$NHCONHCH_3$ | Cl | Cl | Smp. 234—238° |

*Formulierungsbeispiele*

Die Verbindungen der Formel I werden im allgemeinen nicht als solche in der Landwirtschaft eingesetzt. Man verwendet gebrauchsfertige formulierte Mittel, welche entweder direkt oder mit Wasser verdünnt eingesetzt werden können.

Beispiel 7

Stäubemittel

Zur Herstellung eines a) 5%igen und b) 2%igen Stäubemittels werden die folgenden Stoffe verwendet:

a) 5 Teile 2-para-Tolyl-4,6-bis-isopropyloxy-pyrimidin oder einer Mischung davon mit 2-Chlor-2',6'-diäthyl-N-(butoxymethyl)-acetanilid,
95 Teile Talkum,

b) 2 Teile des obigen Wirkstoffes oder einer Mischung,
1 Teil hochdisperse Kieselsäure,
97 Teile Talkum.

Die Wirkstoffe werden mit den Trägerstoffen vermischt und vermahlen und können in dieser Form zur Anwendung verstäubt werden.

Beispiel 8

Granulat

Zur Herstellung eines 5%igen Granulates werden die folgenden Stoffe verwendet:

5 Teile 2-para-Tolyl-4,6-bis-isopropoxy-5-brom-pyrimidin oder einer Mischung davon mit 2-Chlor-2',6'-diäthyl.N-(methoxymethyl)-acetanilid,
0,25 Teile epoxidiertes Pflanzenöl,
0,25 Teile Cetylpolyglykoläther,
3,50 Teile Polyäthylenglykol,
91 Teile Kaolin (Korngrösse 0,3—0,8 mm).

Die Aktivsubstanz oder die Mischung wird mit dem Pflanzenöl vermischt und mit 6 Teilen Aceton gelöst, hierauf wird Polyäthylenglykol und Cetylpolyglykoläther zugesetzt. Die so erhaltene Lösung wird auf Kaolin aufgesprüht, und anschliessend wird das Aceton im Vakuum verdampft. Ein derartiges Mikrogranulat lässt sich vorteilhaft in Saatfurchen einarbeiten.

Beispiel 9

Spritzpulver

Zur Herstellung eines a) 70%igen, b) 40%igen, c) und d) 25%igen, e) 10%igen Spritzpulvers werden folgende Bestandteile verwendet:

a) 70 Teile 2-para-Tolyl-4,6-bis-(methoxyäthyl)-5-chlorpyrimidin oder einer Mischung davon mit 2-Chloro-2',6'-diäthyl-N-(2"-propoxyäthyl)-acetanilid,
5 Teile Natriumdibutylnaphthylsulfonat,
3 Teile Naphthalinsulfonsäuren-Phenolsulfonsäuren-Formaldehyd-Kondensat 3:2:1,
10 Teile Kaolin,
12 Teile Champagne-Kreide;

b) 40 Teile Wirkstoff oder Mischung wie oben,
5 Teile Ligninsulfonsäure-Natriumsalz,
1 Teil Dibutylnaphthalinsulfonsäure-Natriumsalz,
54 Teile Kieselsäure;

c) 25 Teile Wirkstoff oder Mischung wie oben,
4,5 Teile Calcium-Ligninsulfonat,
1,9 Teile Champagne-Kreide/Hydrixyäthylcellulose-Gemisch (1:1),
1,5 Teile Natrium-dibutyl-naphthalinsulfonat,
19,5 Teile Kieselsäure,
19,5 Teile Champagne-Kreide,
28,1 Teile Kaolin;

d) 25 Teile Wirkstoff oder Mischung wie oben,
2,5 Teile Isooctylphenoxy-polyoxyäthylenäthanol,
1,7 Teile Champagne-Kreide/Hydroxyäthylcellulose-Gemisch (1:1),
8,3 Teile Natriumaluminiumsilikat,

16,5 Teile Kieselgur,
46 Teile Kaolin;

e) 10 Teile Wirkstoff oder Mischung wie oben,
3 Teile Gemisch der Natriumsalze von gesättigten Fettalkoholsulfaten,
5 Teile Naphthalinsulfonsäure/Formaldehyd-Kondensat,
82 Teile Kaolin.

Die Wirkstoffe werden in geeigneten Mischern mit den Zuschlagstoffen innig vermischt und auf entsprechenden Mühlen und Walzen vermahlen. Man erhält Spritzpulver von verzüglicher Benetzbarkeit und Schwebefähigkeit, die sich mit Wasser zu Suspensionen der gewünschten Konzentration verdünnen und insbesondere zur Blattapplikation (zwecks Wuchsverzögerung oder für Fungizideinsatz) verwenden lassen.

Beispiel 10

Emulgierbare Konzentrate

Zur Herstellung eines 25%igen emulgierbaren Konzentrates werden folgende Stoffe verwendet:

25 Teile 2-Phenyl-4-chlor-6-methyl-pyrimidin oder einer Mischung davon mit 2-Chlor-6′-äthyl-N-(2″-methoxy-1″-methyläthyl)acet-o-toluidid,
10 Teile eines Alkylarylsulfonat/Fettalkoholpolyglykoläther-Gemisches,
5 Teile Dimethylformamid,
57,5 Teile Xylol.

Beispiel 11

Paste

Zur Herstellung einer 45%igen Paste werden folgende Stoffe verwendet:

a) 45 Teile 2-Phenyl-4-chlor-6-hydroxy-pyrimidin oder einer Mischung davon mit 2-Chlor-2′,6′-diäthyl-N-(methoxymethyl)acetanilid,
5 Teile Natriumaluminiumsilikat,
14 Teile Cetylpolyäthylenglykoläther mit 8 Mol Aethylenoxid,
1 Teil Oleylpolyäthylenglykoläther mit 5 Mol Aethylenoxid,
2 Teile Spindelöl,
23 Teile Wasser,
10 Teile Polyäthylenglykol;

b) 45 Teile des obigen Wirkstoffes oder der Mischung,
5 Teile Aethylenglykol,
3 Teile Octylphenoxypolyäthylenglykol mit 9—10 Mol Aethylenoxid pro Mol Octylphenol,
3 Teile von einem Gemisch aromatischer Sulfonsulfosäuren, kondensiert mit Formaldehyd als Ammoniumsalz,
1 Teil Siliconöl in Form einer 75%igen Emulsion,
0,1 Teile einer Mischung von 1-(3-Chlorallyl)-3,5,7-triazoazonium-adamantan-chlorid mit Natriumcarbonat, Chloridwert mind. 11,5%,
0,2 Teile eines biopolymeren Verdickers mit max. 100 Keimen pro Gramm,
42,7 Teile Wasser.

Die Aktivsubstanz wird mit den Zuschlagstoffen in dazu geeigneten Geräten innig vermischt und vermahlen. Man erhält eine Paste, aus der sich durch Verdünnen mit Wasser Suspensionen jeder gewünschten Konzentration herstellen lassen.

*Biologische Beispiele*

Die Fähigkeit der Verbindungen der Formel I, Kulturpflanzen vor der phytotoxischen Wirkung starker Herbizide zu schützen, kann aus den folgenden Beispielen ersehen werden. In den Versuchsbeschreibungen werden die Verbindungen der Formel I als Antidote (Gegenmittel) bezeichnet. Die Schutzwirkung ist in % angegeben. 0% bedeutet die Wirkung des Herbizides wenn allein appliziert; 100% bedeutet das angestrebte normale Wachstum der Kulturpflanze. Signifikant ist eine Schutzwirkung von mindestens 10%.

Beispiel 12

Versuche mit Antidote und Herbizid in verpflanztem Reis.

Applikationsmethode: *Tankmischung*

Reispflanzen werden bis zum $1\frac{1}{2}$—2 Blattstadium in Erde angezogen. Die Pflanzen werden dann büschelweise (immer 3 Pflanzen zusammen) in Container (47 cm lang, 29 cm breit und 24 cm hoch) in sandigen Lehm verpflanzt. Die Bodenoberfläche wird anschliessend mit Wasser von 1,5—2 cm Höhe

beschichtet. 2—3 Tage nach dem Verpflanzen wird das Herbizid zusammen mit der als Antidote zu prüfenden Substanz als Tankmischung direkt ins Wasser appliziert. 24 Tage nach dem Verpflanzen wird die Schutzwirkung des Antidots in Prozent bonitiert. Als Referenzen dienen dabei die mit Herbizid allein behandelten Pflanzen, (keine Schutzwirkung) sowie die vollständig unbehandelte Kontrolle (= 100% Wachstum). Die Resultate sind untenstehend zusammengefasst.

*Herbizid:* 2-Chlor-2'.6'-diäthyl-N-(2"-propyloxyäthyl)acetanilid ("Pretilachlor"

| Antidote Verbindung Nr. | Aufwandmenge kg/ha | Herbizid Aufwandmengen kg/ha | Schutzwirkung in % |
|---|---|---|---|
| 1 | 1 | 1 | 50 |
| 2 | 0.75 | 0.75 | 25 |
| 3 | 0.75 | 0.75 | 12.5 |
| 4 | 1 | 1 | 12.5 |
| 5 | 1.5 | 1.5 | 25 |
| 10 | 1 | 1 | 25 |
| 30 | 1 | 1 | 25 |
| 43 | 1 | 1 | 25 |

Beispiel 13

*Herbizid:* 2-Chlor-2'.6'-diäthyl-N-(butoxymethyl)acetanilid ("Butachlor"

| Antidote Verbindung Nr. | Aufwandmenge kg/ha | Herbizid Aufwandmengen kg/ha | Schutzwirkung in % |
|---|---|---|---|
| 2 | 1.5 | 1.5 | 12.5 |
| 3 | 1.5 | 1.5 | 12.5 |
| 4 | 1.5 | 1.5 | 25 |
| 5 | 1.5 | 1.5 | 12.5 |
| 6 | 1.5 | 1.5 | 12.5 |

*Herbizid:* 2-Chlor-2',6'-diäthyl-N-(methoxymethyl)acetanilid ("Alachlor"

| Antidote Verbindung Nr. | Aufwandmenge kg/ha | Herbizid Aufwandmengen kg/ha | Schutzwirkung in % |
|---|---|---|---|
| 1 | 0.125 | 0.125 | 12 |
| 2 | 0.125 | 0.125 | 25 |
| 3 | 0.125 | 0.126 | 25 |

*Herbizid:* 2-Chlor-6'-äthyl-N-(äthoxymethyl)cet-o-toluidid "Acetochlor"

| Antidote Verbindung Nr. | Aufwandmenge kg/ha | Herbizid Aufwandmengen kg/ha | Schutzwirkung in % |
|---|---|---|---|
| 2 | 0.125 | 0.125 | 12.5 |
| 3 | 0.125 | 0.125 | 12.5 |

*Herbizid:* 2,6-Dinitro-N,N-dipropyl-4-trifluormethylanilin "Trifluralin"

| Antidote Verbindung Nr. | Aufwandmenge kg/ha | Herbizid Aufwandmengen kg/ha | Schutzwirkung in % |
|---|---|---|---|
| 2 | 2 | 2 | 12.5 |

*Herbizid:* S-4-Chlorbenzyl-diäthylthiocarbamat "Thiobencarb"

| Antidote Verbindung Nr. | Aufwandmenge kg/ha | Herbizid Aufwandmengen kg/ha | Schutzwirkung in % |
|---|---|---|---|
| 1 | 8 | 8 | 50 |
| 1 | 4 | 4 | 25 |
| 1 | 2 | 2 | 12,5 |

Ebenso wird Reis vor der phytotoxischen Wirkung von S-Benzyl-N,N-diäthylthiocarbamat geschützt.

Beispiel 14

Versuche mit Antidote und Herbizid in verpflanztem Reis.

Applikationsmethode- *Wurzelbehandlung*

Reispflanzen der Sorte Yamabiko werden bis 1½—2 Blattstadium in Erde angezogen und dann ausgewaschen. Die Pflanzen werden dann büschelweise (immer 3 Pflanzen zusammen) nur mit den Wurzeln während 15 bis 60 Minuten in eine Schale mit einer Lösung der als Antidote zu prüfenden Substanz von 1000 ppm getaucht. Anschliessend werden sie in Container (47 cm lang, 29 cm breit und 24 cm hoch) in sandigen Lehm verpflanzt. Die Bodenoberfläche wird mit Wasser von 1,5—2 cm Höhe beschichtet. 2—3 Tage nach dem Verpflanzen wird das Herbizid direkt ins Wasser appliziert. 24 Tage nach dem Verpflanzen wird die Schutzwirkung des Antidots in Prozent bonitiert. Als Referenzen dienen dabei die mit Herbizid allein behandelten Pflanzen (keine Schutzwirkung), sowie die vollständig unbehandelte Kontrolle (100% Wachstum). Das Ergebnis ist wie folgt:

*Herbizid:* 2-Chlor-2',6'-diäthyl-N-(2"-propyloxyäthyl)acetanilid "Pretilachlor"

| Antidote Verbindung Nr. | Aufwandmenge | Herbizid Aufwandmengen kg/ha | Schutzwirkung in % |
|---|---|---|---|
| 1 | 1000 ppm | 0.75 | 87.5 |
| 2 | 1000 ppm | 1 | 50 |

Beispiel 15

Versuch mit Antidote und Herbizid in verpflanztem Reis.

Applikation der Antidote auf die Pflanze mittels wässriger Lösung (Drench-Methode).

Reispflanzen der Sorte Yamabiko werden bis zum 1½—2 Blattstadium in Anzuchtschalen angezogen. 1—2 Tage vor dem Verpflanzen wird die ganze Anzuchtschale mit den Reispflanzen in einer grösseren

Schale, welche eine Lösung mit der als Antidote zu prüfenden Substanz in einer Konzentration von 1000 ppm enthält, getaucht. Die Pflanzen werden dann büschelweise (immer 3 Pflanzen) in Container (47 cm lang, 29 cm breit und 24 cm hoch) in sandigen Lehm verpflanzt. Die Bodenfläche wird mit Wasser 1,5—2 cm Höhe beschichtet. 2—3 Tage nach dem Verpflanzen wird das Herbizid direkt ins Wasser appliziert. 24 Tage nach dem Verpflanzen wird die Schutzwirkung des Antidots in Prozent bonitiert. Als Referenzen dienen dabei die mit Herbizid allein behandelten Pflanzen (keine Schutzwirkung), sowie die vollständig unbehandelte Kontrolle (100% Wachstum). Das Ergebnis ist wie folgt:

*Herbizid:* 2-Chlor-2',6'-diäthyl-N-(2"-propyloxyäthyl)acetanilid "Pretilachlor"

| Antidote Verbindung Nr. | Aufwandmenge kg/ha | Herbizid Aufwandmengen kg/ha | Schutzwirkung in % |
|---|---|---|---|
| 2 | 1000 ppm | 1 | 37.5 |

Beispiel 16

Versuch mit Antidote und Herbizid in verpflanztem Reis.

Applikation der Antidote im Vorauflaufverfahren.

Die als Antidote zu prüfende Substanz wird als verdünnte Lösung auf die feuchte Erdoberfläche von Anzuchtschalen versprüht. Anschliessend werden Reissamen eingesät und bis zum 1½—2 Blattstadium angezogen. Die Pflanzen werden dann (immer 3 Pflanzen zusammen) in Container (47 cm lang, 29 cm breit und 24 cm hoch) in sandigen Lehm verpflanzt. Die Bodenoberfläche wird mit Wasser von 1,5—2 cm Höhe beschichtet. 2—3 Tage nach dem Verpflanzen wird das Herbizid direkt ins Wasser appliziert und 24 Tage nach dem Verpflanzen wird die Schutzwirkung des Antidots in Prozent bonitiert. Als Referenzen dienen dabei die mit Herbizid allein behandelten Pflanzen (keine Schutzwirkung) sowie die vollständig unbehandelte Kontrolle (100% Wachstum). Das Ergebnis ist wie folgt:

*Herbizid:* 2-Chlor-2'.6'-diäthyl-N-(2"-propyloxyäthyl)acetanilid "Pretolachlor"

| Antidote Verbindung Nr. | Aufwandmenge kg/ha | Herbizid Aufwandmengen kg/ha | Schutzwirkung in % |
|---|---|---|---|
| 2 | 4 | 1.5 | 25 |

Beispiel 17

Versuch mit Antidote und Herbizid in verpflanztem Reis.

Applikation der Antidote durch Einmischen in den Boden (ppi-Methode).

Die als Antidote zu prüfende Substanz wird in die Erde in Anzuchtschalen in einer Konzentration von 100 ppm eingemischt. 2 Tage später werden die Reispflanzen bis zum 1½—2 Blattstadium in den behandelten Anzuchtschalen angezogen. Die Pflanzen werden dann büschelweise (immer 3 Pflanzen zusammen) in Container (47 cm lang, 29 cm breit und 24 cm hoch) in sandigen Lehm verpflanzt. Die Bodenoberfläche wird mit Wasser von 1,5—2 cm Höhe beschichtet. 2—3 Tage nach dem Verpflanzen wird das Herbizid direkt ins Wasser appliziert. 24 Tage nach dem Verpflanzen wird die Schutzwirkung des Antidots in Prozent bonitiert. Als Referenzen dienen dabei die mit Herbizid allein behandelten Pflanzen (keine Schutzwirkung) sowie die vollständig unbehandelte Kontrolle (100% Wachstum). Die Ergebnisse sind wie folgt:

*Herbizid:* 2-Chlor-2'.6'-diäthyl-N-(2"-propyloxyäthyl)acetanilid "Pretilachlor"

| Antidote Verbindung Nr. | Aufwandmenge | Herbizid Aufwandmenge kg/ha | Schutzwirkung in % |
|---|---|---|---|
| 2 | 100 ppm | 1 | 25 |
| 2 | 100 ppm | 0.75 | 25 |

Beispiel 18

Versuch mit Antidote und Herbizid in verpflanztem Reis.

Applikation der Antidote im Nachauflaufverfahren (over the top-Applikation)

Reispflanzen der Sorte Yamabiko werden bis zum 1½—2 Blattstadium in Erde angezogen. Zu diesem Zeitpunkt wird die als Antidote zu prüfende Substanz als verdünnte Lösung über den Reispflanzen versprüht. Zwei Tage später werden die Pflanzen büschelweise (immer 3 Pflanzen zusammen) in Container

(47 cm lang, 29 cm breit und 24 cm hoch) in sandigen Lehm verpflanzt. Die Bodenoberfläche wird mit Wasser von 1,5—2 cm Höhe beschichtet. 2—3 Tage nach dem Verpflanzen wird das Herbizid direkt ins Wasser appliziert. 24 Tage nach dem Verpflanzen wird die Schutzwirkung des Antidots in Prozent bonitiert. Als Referenzen dienen dabei die mit Herbizid allein behandelten Pflanzen (keine Schutzwirkung) sowie die vollständig unbehandelte Kontrolle (100% Wachstum). Das Ergebnis ist wie folgt:

*Herbizid:* 2-Chlor-2'.6'-diäthyl-N-(2"-propyloxyäthyl)acetanilid "Pretilachlor"

| Antidote Verbindung Nr. | Aufwandmenge kg/ha | Herbizid Aufwandmenge kg/ha | Schutzwirkung in % |
|---|---|---|---|
| 2 | 4 | 1.5 | 12 |

Die erfindungsgemässen 2-Phenylpyrimidine bewirkten in den in den Beispielen 13 bis 18 beschriebenen Methoden eine gewisse Schutzwirkung auf den verpflanzten Reis auch dann wenn anstelle der oben erwähnten, die Herbizide S-2-Methylpiperidino-carbonylmethyl-O,O-dipropylphosphorodithioate ("Piperophos"), S-Aethyl-N,N-hexamethylen-thiocarbamat ("Molinate"), S-4-Chlorbenzyl-diäthyl-thiocarbamat ("Thiobencarb"), S-4-Benzyl-diäthyl-thiocarbamat, 5-tert.Butyl-3-(2,4-dichlor-5-isopropoxyphenyl)-1,3,4-oxadiazol-2-on ("Oxadiazon") oder N-(3,4-Dichlorphenyl-propionamid ("Propanil") verwendet werden.

Beispiel 19

Versuch mit Antidote und Herbizid mit in Wasser gesätem Reis. (Die Reissamen werden vorgequollen und direkt auf sehr nasse, sumpfartige oder überflutete Böden gesät.)
Applikation der Antidote als Tankmischung.

Reissamen werden während 48 Stunden in Wasser vorgequollen. Plastik-Container (25 cm lang, 17 cm breit und 12 cm hoch) werden mit Erde gefüllt, in die die vorgequollenen Reissamen eingesät werden. Anschliessend wird die als Antidote zu prüfende Substanz zusammen mit dem Herbizid als Tankmischung versprüht. Der Wasserstand wird entsprechend dem Wachstum der Reispflanzen sukzessive erhöht. 21 Tage danach wird die Schutzwirkung des Antidots in Prozent bonitiert. Als Referenzen dienen dabei die mit Herbizid allein behandelten Pflanzen (keine Schutzwirkung) sowie die vollständig unbehandelte Kontrolle (100% Wachstum). Die Ergebnisse sind in den untenstehenden Tabellen zusammengefasst.

*Herbizid:* 2-Chlor-2'.6'-diäthyl-N-(2"-propyloxyäthyl)acetanilid "Pretilachlor"

| Antidote Verbindung Nr. | Aufwandmenge kg/ha | Herbizid Aufwandmenge kg/ha | Schutzwirkung in % |
|---|---|---|---|
| 1 | 0.25 | 0.25 | 62.5 |
| 2 | 0.25 | 0.25 | 50 |
| 3 | 0.25 | 0.25 | 37.5 |
| 4 | 0.25 | 0.25 | 50 |
| 5 | 0.25 | 0.25 | 62.5 |
| 6 | 0.25 | 0.25 | 62.5 |
| 9 | 0.25 | 0.25 | 50 |
| 10 | 0.25 | 0.25 | 75 |
| 15 | 0.25 | 0.25 | 12.5 |
| 17 | 0.25 | 0.25 | 37.5 |
| 30 | 0.25 | 0.25 | 75 |
| 34 | 0.25 | 0.25 | 25 |
| 43 | 0.25 | 0.25 | 62.5 |

| Antidote Verbindung Nr. | Aufwandmenge kg/ha | Herbizid Aufwandmenge kg/ha | Schutzwirkung in % |
|---|---|---|---|
| 68 | 0.25 | 0.25 | 37.5 |
| 69 | 0.25 | 0.25 | 62.5 |
| 71 | 0.25 | 0.25 | 37.5 |
| 73 | 0.25 | 0.25 | 62.5 |
| 74 | 0.25 | 0.25 | 50 |
| 87 | 0.25 | 0.25 | 50 |
| 110 | 0.25 | 0.25 | 25 |
| 111 | 0.25 | 0.25 | 62.5 |
| 113 | 0.25 | 0.25 | 50 |
| 120 | 0.25 | 0.25 | 25 |
| 130 | 0.25 | 0.25 | 62.5 |
| 236 | 0.25 | 0.25 | 62.5 |
| 279 | 0.25 | 0.25 | 25 |
| 334 | 0.25 | 0.25 | 37.5 |
| 341 | 0.25 | 0.25 | 25 |
| 364 | 0.25 | 0.25 | 75 |

*Herbizid:* 2-Chlor-2'.6'-diäthyl-N-(butoxymethyl)acetanilid "Butachlor"

| Antidote Verbindung Nr. | Aufwandmenge kg/ha | Herbizid Aufwandmenge kg/ha | Schutzwirkung in % |
|---|---|---|---|
| 1 | 0.5 | 0.15 | 37.5 |
| 2 | 0.75 | 0.75 | 50 |
| 3 | 0.75 | 0.75 | 50 |
| 4 | 0.75 | 0.75 | 37.5 |
| 5 | 0.75 | 0.75 | 62.5 |
| 6 | 0.75 | 0.75 | 62.5 |

*Herbizid:* 2-Chlor-2',6'-diäthyl-N-(methoxymethyl)acetanilid "Alachlor"

| Antidote Verbindung Nr. | Aufwandmenge kg/ha | Herbizid Aufwandmenge kg/ha | Schutzwirkung in % |
|---|---|---|---|
| 1 | 0.03 | 0.03 | 25 |
| 2 | 0.03 | 0.03 | 12.5 |
| 3 | 0.03 | 0.03 | 37.5 |
| 4 | 0.03 | 0.03 | 37.5 |
| 5 | 0.03 | 0.03 | 25 |
| 6 | 0.03 | 0.03 | 37.5 |

*Herbizid:* 2-Chlor-6'-äthyl-N-(2"-methoxy-1"-methyläthyl)acet-o-toluidid, "Metolachlor"

| Antidote Verbindung Nr. | Aufwandmenge kg/ha | Herbizid Aufwandmenge kg/ha | Schutzwirkung in % |
|---|---|---|---|
| 1 | 0.03 | 0.03 | 25 |
| 2 | 0.03 | 0.03 | 12.5 |
| 3 | 0.03 | 0.03 | 25 |
| 4 | 0.03 | 0.03 | 12.5 |
| 5 | 0.03 | 0.03 | 25 |
| 6 | 0.03 | 0.03 | 12.5 |

*Herbizid:* S-ethyl-diisopropyl-thiocarbamat, "EPTC"

| Antidote Verbindung Nr. | Aufwandmenge kg/ha | Herbizid Aufwandmenge kg/ha | Schutzwirkung in % |
|---|---|---|---|
| 1 | 1 | 1 | 25 |

*Herbizid:* S-2-Methylpiperidino-carbonylmethyl-O,O-dipropyl-phosphothioat, "Piperophos"

| Antidote Verbindung Nr. | Aufwandmenge kg/ha | Herbizid Aufwandmenge kg/ha | Schutzwirkung in % |
|---|---|---|---|
| 1 | 0.5 | 0.5 | 37.5 |

*Herbizid:* 5-Aethyl-N,N-hexamethylen-thiocarbamat "Molinate"

| Antidote Verbindung Nr. | Aufwandmenge kg/ha | Herbizid Aufwandmenge kg/ha | Schutzwirkung in % |
|---|---|---|---|
| 1 | 2 | 2 | 25 |

*Herbizid:* S-4-Chlorbenzyl-diäthylthiocarbamat, "Thiobencarb"

| Antidote Verbindung Nr. | Aufwandmenge kg/ha | Herbizid Aufwandmenge kg/ha | Schutzwirkung in % |
|---|---|---|---|
| 1 | 4 | 4 | 37.5 |

Das Antidote, Verbindung Nr. 1, aber auch andere erfindungsgemässe 2-Phenyl-pyrimidine vermochten in diesem Versuch den Reis auch gegen die phytotoxische Wirkung von S-4-Benzyl-diäthylthiocarbamat und 5-tert.-Butyl-3-(2,4-dichlor-5-isopropoxyphenyl)-1,3,4-oxdiazol-2-on ("Oxdiazon"), zu schützen.

Beispiel 20

Versuch mit Antidote und Herbizid mit in Wasser gesätem Reis.
Applikation der Antidote während der Samenquellung des Reises.

Reissamen werden während 48 Stunden mit Lösungen der als Antidote zu prüfenden Substanz von 100 ppm getränkt. Anschliessend werden die Samen etwa 2 Stunden trocknen gelassen, bis sie nicht mehr kleben. Plasik Container (25 cm lang, 17 cm breit und 12 cm hoch) werden bis 2 cm unter dem Rand mit sandigem Lehm gefüllt. Die vorgequollenen Samen werden auf der Bodenfläche des Containers gesät und nun ganz schwach gedeckt. Die Erde wird in einem feuchten (nicht sumpfigen) Zustand gehalten. Dann wird das Herbizid in verdünnter Lösung auf die Bodenoberfläche versprüht. Der Wasserstand wird entsprechend dem Wachstum sukzessive erhöht. 21 Tage danach wird die Schutzwirkung des Antidots in Prozent bonitiert. Als Referenzen dienen dabei die mit dem Herbizid allein behandelten Pflanzen (keine Schutzwirkung) sowie die vollständig unbehandelte Kontrolle (100% Wachstum). Die Ergebnisse sind in den untenstehenden Tabellen zusammengefasst.

*Herbizid:* 2-Chlor-2',6'-diäthyl-N-(2"-propyloxyäthyl)acetanilid "Pretilachlor"

| Antidote Verbindung Nr. | Aufwandmenge | Herbizid Aufwandmenge kg/ha | Schutzwirkung in % |
|---|---|---|---|
| 1 | 100 ppm | 0.25 | 62.5 |
| 2 | 100 ppm | 0.25 | 75 |
| 3 | 100 ppm | 0.25 | 75 |
| 4 | 100 ppm | 0.25 | 87.5 |
| 5 | 100 ppm | 0.25 | 62.5 |
| 6 | 100 ppm | 0.25 | 62.5 |
| 9 | 100 ppm | 0.25 | 50 |
| 10 | 100 ppm | 0.25 | 62.5 |
| 15 | 100 ppm | 0.25 | 50 |
| 17 | 100 ppm | 0.25 | 50 |
| 30 | 100 ppm | 0.25 | 50 |
| 34 | 100 ppm | 0.25 | 62.5 |
| 43 | 100 ppm | 0.25 | 62.5 |
| 67 | 100 ppm | 0.25 | 50 |
| 69 | 100 ppm | 0.25 | 50 |
| 71 | 100 ppm | 0.25 | 37.5 |
| 73 | 100 ppm | 0.25 | 50 |

*Herbizid:* 2-Chlor-2',6'-diäthyl-N-(2"-propyloxyäthyl)acetanilid "Pretilachlor"

| Antidote Verbindung Nr. | Aufwandmenge | Herbizid Aufwandmenge kg/ha | Schutzwirkung in % |
|---|---|---|---|
| 74 | 100 ppm | 0.25 | 50 |
| 87 | 100 ppm | 0.25 | 75 |
| 110 | 100 ppm | 0.25 | 62.5 |
| 111 | 100 ppm | 0.25 | 75 |
| 113 | 100 ppm | 0.25 | 75 |
| 120 | 100 ppm | 0.25 | 62.5 |
| 130 | 100 ppm | 0.25 | 25 |
| 236 | 100 ppm | 0.25 | 50 |
| 279 | 100 ppm | 0.25 | 50 |
| 334 | 100 ppm | 0.25 | 62.5 |
| 341 | 100 ppm | 0.25 | 75 |
| 364 | 100 ppm | 0.25 | 37.5 |

*Herbizid:* 2-Chlor-2',6'-diäthyl-N-(butoxymethyl)acetanilid "Butachlor"

| Antidote Verbindung Nr. | Aufwandmenge | Herbizid Aufwandmenge kg/ha | Schutzwirkung in % |
|---|---|---|---|
| 1 | 100 ppm | 0.5 | 50 |
| 2 | 100 ppm | 0.5 | 50 |
| 3 | 100 ppm | 0.5 | 37.5 |
| 4 | 100 ppm | 0.5 | 50 |
| 5 | 100 ppm | 0.5 | 50 |
| 6 | 100 ppm | 0.5 | 50 |

*Herbizid:* 2-Chlor-2',6'-diäthyl-N-(methoxyäthyl)acetanilid "Alachlor"

| Antidote Verbindung Nr. | Aufwandmenge | Herbizid Aufwandmenge kg/ha | Schutzwirkung in % |
|---|---|---|---|
| 1 | 100 ppm | 0.03 | 50 |
| 2 | 100 ppm | 0.03 | 62.5 |
| 3 | 100 ppm | 0.03 | 50 |
| 4 | 100 ppm | 0.03 | 62.5 |
| 5 | 100 ppm | 0.03 | 62.5 |
| 6 | 100 ppm | 0.03 | 50 |

*Herbizid:* 2-Chlor-6'-äthyl-N-(2"-methoxy-1"-methyläthyl)acet-o-toluidid "Metolachlor"

| Antidote Verbindung Nr. | Aufwandmenge | Herbizid Aufwandmenge kg/ha | Schutzwirkung in % |
|---|---|---|---|
| 2 | 100 ppm | 0.03 | 12.5 |
| 3 | 100 ppm | 0.03 | 12.5 |

*Herbizid:* S-Ethyl-diisopropyl-thiocarbamat, "EPTC"

| Antidote Verbindung Nr. | Aufwandmenge | Herbizid Aufwandmenge kg/ha | Schutzwirkung in % |
|---|---|---|---|
| 1 | 100 ppm | 0.5 | 12.5 |

*Herbizid:* S-2-Methylpiperidino-carbonylmethyl-O,O-dipropyl-phosphoro-dithioate, "Piperophos"

| Antidote Verbindung Nr. | Aufwandmenge | Herbizid Aufwandmenge kg/ha | Schutzwirkung in % |
|---|---|---|---|
| 1 | 1000 ppm | 6.5 | 37.5 |
| 1 | 100 ppm | 6.5 | 37.5 |
| 1 | 10 ppm | 6.5 | 25 |

*Herbizid:* S-Aethyl-N,N-hexamethylen-thiocarbamat "Molinate"

| Antidote Verbindung Nr. | Aufwandmenge | Herbizid Aufwandmenge kg/ha | Schutzwirkung in % |
|---|---|---|---|
| 1 | 1000 ppm | 8 | 62.5 |
| 1 | 100 ppm | 8 | 62.5 |
| 1 | 10 ppm | 8 | 62.5 |
| 1 | 1000 ppm | 2 | 37.5 |
| 1 | 100 ppm | 2 | 37.5 |
| 1 | 10 ppm | 2 | 37.5 |

*Herbizid:* S-4-Chlorbenzyl-diäthylthiocarbamat "Thiocarb"

| Antidote Verbindung Nr. | Aufwandmenge | Herbizid Aufwandmenge kg/ha | Schutzwirkung in % |
|---|---|---|---|
| 1 | 1000 ppm | 8 | 37.5 |
| 1 | 100 ppm | 8 | 37.5 |
| 1 | 1000 ppm | 4 | 50 |
| 1 | 100 ppm | 4 | 50 |
| 1 | 10 ppm | 4 | 37.5 |
| 1 | 1000 ppm | 2 | 37.5 |
| 1 | 100 ppm | 2 | 25 |
| 1 | 10 ppm | 2 | 25 |

Ebenso wurde Reis vor der phytotoxischen Wirkung von S-Benzyl-diäthyl-thiocarbamat und 5-tert.Butyl-3-(2,4-dichlor-5-isopropoxyphenyl)-1,3,4-oxdiazol-2-on ("Oxdiazon") geschützt.

Beispiel 21

Versuch mit Antidote und Herbizid mit in Wasser gesätem Reis.

Applikation von Antidote und Herbizid in Nährlösung.

Man verwendete Reissamen, die in den eingesetzten Konzentrationen des Herbizides erwartungsgemäss geschädigt werden sollten und sät sie in gekörntes Zonolith (= expandierter Vermikulit), das sich in einem an der Unterseite durchlöcherten Plastik-Blumentopf (oberer Durchmesser 6 cm) befindet. Dieser wird in einen zweiten durchsichtigen Plastik-Blumentopf (oberer Durchmesser 7 cm) gestellt, in den sich ca. 50 ml der mit Herbizid und Antidote vorbereiteten Nährlösung befindet, die nunmehr kapillar im Filtermaterial des kleineren Topfes aufsteigt und Samen und keimende Pflanzen benetzt. Täglich wird der Flüssigkeitsverlust mit einer Hewitt-Nährlösung auf 50 ml ergänzt. 3 Wochen nach Testbeginn wird die Schutzwirkung des Antidots in Prozent bonitiert. Als Referenzen dienen dabei die mit Herbizid allein behandelten Pflanzen (keine Schutzwirkung) sowie die vollständig unbehandelte Kontrolle (100% Wachstum). Das Ergebnis ist wie folgt:

*Herbizid:* 2-Chlor-2',6'-diäthyl-N-(2"-propyloxyäthyl)acetanilid "Pretilachlor"

| Antidote Verbindung Nr. | Aufwandmenge | Herbizid Aufwandmenge | Schutzwirkung in % |
|---|---|---|---|
| 1 | 10 ppm | 4 ppm | 75 |

Beispiel 22

Versuch mit Antidote und Herbizid mit trocken gesätem Reis (20 Tage nach der Saat, wenn die Reispflanzen das 3-Blatt Stadium erreicht haben, wird der Boden überflutet).

Applikation von Antidote und Herbizid als Tankmischung.

Reissamen der Sorte IR—36 werden in Container (47 cm lang, 29 cm breit und 24 cm hoch) eingesät, bedeckt und leicht festgedrückt. Dann wird die als Antidote zu prüfende Substanz zusammen mit dem Herbizid als Tankmischung versprüht. Etwa 20 Tage nach der Saat (3-Blattstadium der Reispflanzen) wird eine Bodenoberfläche mit 4 cm Wasser Höhe beschichtet. 30 Tage nach dem Verpflanzen wird die Schutzwirkung des Antidots in Prozent bonitiert. Als Referenzen dienen dabei die mit Herbizid allein behandelten Pflanzen (keine Schutzwirkung) sowie die collständig unbehandelte Kontrolle (100% Schutzwirkung). Die Ergebnisse sind in den folgenden Tabellen festgehalten.

*Herbizid:* 2-Chlor-2',6'-diäthyl-N-(2"-propyloxyäthyl)acetanilid "Pretilachlor"

| Antidote Verbindung Nr. | Aufwandmenge kg/ha | Herbizid Aufwandmenge kg/ha | Schutzwirkung in % |
|---|---|---|---|
| 1 | 2 | 2 | 62.5 |
| 2 | 2 | 2 | 50 |
| 3 | 2 | 2 | 50 |
| 4 | 2 | 2 | 50 |
| 5 | 2 | 2 | 62.5 |
| 6 | 2 | 2 | 50 |
| 10 | 2 | 2 | 50 |
| 30 | 3 | 3 | 62.5 |
| 43 | 2 | 2 | 27.5 |

*Herbizid:* 2-Chlor-2',6'-diäthyl-N-(butoxymethyl)acetanilid "Butachlor"

| Antidote Verbindung Nr. | Aufwandmenge kg/ha | Herbizid Aufwandmenge kg/ha | Schutzwirkung in % |
|---|---|---|---|
| 1 | 3 | 3 | 25 |
| 2 | 3 | 3 | 25 |
| 3 | 3 | 3 | 37.5 |
| 4 | 3 | 3 | 37.5 |
| 5 | 3 | 3 | 25 |
| 6 | 3 | 3 | 37.5 |

*Herbizid:* 2-Chlor-2',6'-diäthyl-N-(methoxymethyl)acetanilid "Alachlor"

| Antidote Verbindung Nr. | Aufwandmenge kg/ha | Herbizid Aufwandmenge kg/ha | Schutzwirkung in % |
|---|---|---|---|
| 1 | 0.25 | 0.25 | 37.5 |
| 2 | 0.5 | 0.5 | 25 |
| 3 | 0.5 | 0.5 | 12.5 |
| 4 | 0.25 | 0.25 | 25 |
| 5 | 0.25 | 0.25 | 25 |
| 6 | 0.5 | 0.5 | 25 |

*Herbizid:* 2-Chlor-6-äthyl-N-(2"-methoxy-1"-methyläthyl)acet-o-toluidid "Metolachlor"

| Antidote Verbindung Nr. | Aufwandmenge kg/ha | Herbizid Aufwandmenge kg/ha | Schutzwirkung in % |
|---|---|---|---|
| 1 | 3 | 0.75 | 37.5 |

*Herbizid:* 2-Chlor-6-äthyl-N-(äthoxymethyl)acet-o-toluidid "Acetochlor"

| Antidote Verbindung Nr. | Aufwandmenge kg/ha | Herbizid Aufwandmenge kg/ha | Schutzwirkung in % |
|---|---|---|---|
| 1 | 0.25 | 0.25 | 25 |
| 2 | 0.25 | 0.25 | 12.5 |
| 3 | 0.25 | 0.25 | 12.5 |
| 4 | 0.25 | 0.25 | 12.5 |
| 5 | 0.25 | 0.25 | 12.5 |
| 6 | 0.25 | 0.25 | 12.5 |

*Herbizid:* 2,6-Dinitro-N,N-dipropyl-4-trifluormethylanilin "Trifluralin"

| Antidote Verbindung Nr. | Aufwandmenge kg/ha | Herbizid Aufwandmenge kg/ha | Schutzwirkung in % |
|---|---|---|---|
| 1 | 1 | 1 | 37.5 |
| 2 | 1 | 1 | 25 |
| 4 | 1 | 1 | 12.5 |
| 5 | 1 | 1 | 25 |
| 6 | 1 | 1 | 37.5 |

*Herbizid:* 2,6-Dichlorbenzonitril, "Dichlobenil"

| Antidote Verbindung Nr. | Aufwandmenge kg/ha | Herbizid Aufwandmengen kg/ha | Schutzwirkung in % |
|---|---|---|---|
| 1 | 0.5 | 0.5 | 12.5 |
| 2 | 0.5 | 0.5 | 12.5 |
| 3 | 0.5 | 0.5 | 25 |
| 4 | 0.5 | 0.5 | 25 |
| 5 | 0.5 | 0.5 | 25 |
| 6 | 0.5 | 0.5 | 25 |

*Herbizid:* S-2,3,3-Trichlorallyl-di-isopropyl-thiocarbamate "Tri-allate"

| Antidote Verbindung Nr. | Aufwandmenge kg/ha | Herbizid Aufwandmengen kg/ha | Schutzwirkung in % |
|---|---|---|---|
| 1 | 4 | 4 | 12.5 |
| 2 | 4 | 4 | 25 |
| 3 | 4 | 4 | 12.5 |
| 4 | 4 | 4 | 25 |
| 5 | 4 | 4 | 25 |
| 6 | 4 | 4 | 25 |

## Beispiel 23

Versuch mit Antidote und Herbizid mit trocken gesätem Reis.
Applikation der Antidote als Saatbeizung.

Reissamen werden mit der als Antidote zu prüfenden Substanz in einen Glasbehälter gemischt. Samen und Produkt werden durch Schütteln und Rotation gut zusammengemischt. Dann werden Container (47 cm lang, 29 cm breit und 24 cm hoch) mit sandiger Lehmerde gefüllt und die gebeizten Samen werden eingesät. Nach dem Bedecken des Samens wird das Herbizid in einer verdünnten Lösung auf die Bodenoberfläche versprüht. Etwa 20 Tage nach der Saat (3-Blattstadium der Reispflanzen) wird die Bodenoberfläche mit 4 cm Wasser Höhe beschichtet. 30 Tage nach der Herbizidapplikation wird die Schutzwirkung des Antidots in Prozent binitiert. Als Referenzen dienen dabei die mit Herbizid allein behandelten Pflanzen (keine Schutzwirkung) sowie die vollständig unbehandelte Kontrolle (100% Wachstum). Die Ergebnisse sind wie folgt:

*Herbizid:* 2-Chlor-2',6'-diäthyl-N-(2"-propyloxyäthyl)acetanilid "Pretilachlor"

| Antidote Verbindung Nr. | Aufwandmenge g pro kg Saatgut | Herbizid Aufwandmenge kg/ha | Schutzwirkung in % |
|---|---|---|---|
| 1 | 2 g | 3 | 37.5 |

*Herbizid:* 2-Chlor-6'-äthyl-N-(2"-methoxy-1"-methyläthyl)acet-o-toluidid, "Metolachlor"

| Antidote Verbindung Nr. | Aufwandmenge pro kg Saatgut | Herbizid Aufwandmenge kg/ha | Schutzwirkung in % |
|---|---|---|---|
| 1 | 1 g | 0.5 | 62.5 |

## Beispiel 24

Versuch mit Antidote und Herbizid in "Trockenreis". (Der Reis wird trocken gesät, die Bewässerung erfolgt durch natürliche Regenfälle).
Applikation von Antidote und Herbizid als Tankmischung.

Reissamen werden in Container (47 cm lang, 29 cm breit und 24 cm hoch) eingesät, bedeckt und leicht festgedrückt. Dann wird die als Antidote zu prüfende Substanz als verdünnte Lösung zusammen mit dem Herbizid als Tankmischung versprüht. 24 Tage nach der Saat wird die Schutzwirkung des Antidots in Prozent bonitiert. Als Referenzen dienen dabei die mit Herbizid allein behandelten Pflanzen (keine Schutzwirkung) sowie die vollständig unbehandelte Kontrolle (100% Wachstum). Die Ergebnisse sind in den untenstehenden Tabellen zusammengefasst.

*Herbizid:* 2-Chlor-2',6'-diäthyl-N-(2"-propyloxyäthyl)acetanilid "Pretilachlor"

| Antidote Verbindung Nr. | Aufwandmenge kg/ha | Herbizid Aufwandmengen kg/ha | Schutzwirkung in % |
|---|---|---|---|
| 1 | 2 | 2 | 62.5 |
| 2 | 2 | 2 | 50 |
| 3 | 2 | 2 | 50 |
| 4 | 2 | 2 | 50 |
| 5 | 2 | 2 | 62.5 |
| 6 | 2 | 2 | 50 |
| 10 | 2 | 2 | 50 |
| 30 | 2 | 2 | 62.5 |
| 43 | 2 | 2 | 37.5 |

*Herbizid:* 2-Chlor-2',6'-diäthyl-N-(butoxymethyl)acetanilid "Butolachlor"

| Antidote Verbindung Nr. | Aufwandmenge kg/ha | Herbizid Aufwandmengen kg/ha | Schutzwirkung in % |
|---|---|---|---|
| 1 | 3 | 3 | 25 |
| 2 | 3 | 3 | 25 |
| 3 | 3 | 3 | 37.5 |
| 4 | 3 | 3 | 37.5 |
| 5 | 3 | 3 | 25 |
| 6 | 3 | 3 | 37.5 |

*Herbizid:* 2-Chlor-2'.6'-diäthyl-N-(methoxymethyl)acetanilid "Alachlor"

| Antidote Verbindung Nr. | Aufwandmenge kg/ha | Herbizid Aufwandmengen kg/ha | Schutzwirkung in % |
|---|---|---|---|
| 1 | 0.25 | 0.25 | 37.5 |
| 2 | 0.5 | 0.5 | 25 |
| 3 | 0.5 | 0.5 | 12.5 |
| 4 | 0.5 | 0.5 | 25 |
| 5 | 0.25 | 0.25 | 25 |
| 6 | 0.5 | 0.5 | 25 |

*Herbizid:* 2-Chlor-6'-äthyl-N-(2"-methoxy-1"-methyläthyl)acet-o-toluidid, "Metolachlor"

| Antidote Verbindung Nr. | Aufwandmenge kg/ha | Herbizid Aufwandmenge kg/ha | Schutzwirkung in % |
|---|---|---|---|
| 1 | 3 | 0.75 | 37.5 |

*Herbizid:* 2-Chlor-6'-äthyl-N-(äthoxymethyl)acet-o-toluidid "Acetochlor"

| Antidote Verbindung Nr. | Aufwandmenge kg/ha | Herbizid Aufwandmengen kg/ha | Schutzwirkung in % |
|---|---|---|---|
| 1 | 0.25 | 0.25 | 25 |
| 2 | 0.25 | 0.25 | 12.5 |
| 3 | 0.25 | 0.25 | 12.5 |
| 4 | 0.25 | 0.25 | 12.5 |
| 5 | 0.25 | 0.25 | 12.5 |
| 6 | 0.25 | 0.25 | 12.5 |

*Herbizid:* 2,6-Dinitro-N,N-dipropyl-4-trifluormethylanilin, "Trifluralin"

| Antidote Verbindung Nr. | Aufwandmenge kg/ha | Herbizid Aufwandmenge kg/ha | Schutzwirkung in % |
|---|---|---|---|
| 1 | 1 | 1 | 37.5 |
| 2 | 1 | 1 | 25 |
| 4 | 1 | 1 | 12.5 |
| 5 | 1 | 1 | 37.5 |
| 6 | 1 | 1 | 25 |

*Herbizid:* 2,6-Dichlorbenzonitril, "Dichlobenil"

| Antidote Verbindung Nr. | Aufwandmenge kg/ha | Herbizid Aufwandmenge kg/ha | Schutzwirkung in % |
|---|---|---|---|
| 1 | 0.5 | 0.5 | 12.5 |
| 2 | 0.5 | 0.5 | 12.5 |
| 3 | 0.5 | 0.5 | 25 |
| 4 | 0.5 | 0.5 | 25 |
| 5 | 0.5 | 0.5 | 25 |
| 6 | 0.5 | 0.5 | 25 |

*Herbizid:* S-2,3-Dichlorallyl-di-isopropyl-thiocarbamate, "Di-allate"

| Antidote Verbindung Nr. | Aufwandmenge kg/ha | Herbizid Aufwandmenge kg/ha | Schutzwirkung in % |
|---|---|---|---|
| 1 | 4 | 4 | 12.5 |
| 2 | 4 | 4 | 25 |
| 3 | 4 | 4 | 12.5 |
| 4 | 4 | 4 | 25 |
| 5 | 4 | 4 | 25 |
| 6 | 4 | 4 | 25 |

Beispiel 25

Versuch mit Antidote und Herbizid in "Trockenreis".
Applikation der Antidote als Saatbeizung.

Reissamen der Sorte IR—36 werden mit der als Antidote zu prüfenden Substanz in einen Glasbehälter gemischt. Samen und Produkt werden durch Schütteln und Roation gut zusammengemischt. Anschliessend werden Plastikcontainer (47 cm lang, 29 cm breit und 24 cm hoch) mit sandiger Lehmerde gefüllt und die gebeizten Samen werden eingesät. Nach dem Bedecken des Samens wird das Herbizid auf die Bodenoberfläche versprüht. 18 Tage nach der Saat wird die Schutzwirkung des Antidots in Prozent bonitiert. Als Referenzen dienen dabei die mit Herbizid allein behandelten Pflanzen (keine Schutzwirkung) sowie die vollständig unbehandelte Kontrolle (100% Wachstum). Die Ergebnisse sind wie folgt.

*Herbizid:* 2-Chlor-6'-äthyl-N-(2"-methoxy-1"-methyläthyl)acet-o-toluidid, "Metolachlor"

| Antidote Verbindung Nr. | Aufwandmenge g pro kg Saatgut | Herbizid Aufwandmenge kg/ha | Schutzwirkung in % |
|---|---|---|---|
| 1 | 1 g | 0.5 | 62.5 |

*Herbizid:* 2-Chlor-2,6-diäthyl-N-(2"-propyloxyäthyl)acetanilid "Pretilachlor"

| Antidote Verbindung Nr. | Aufwandmenge g pro Saatgut | Herbizid Aufwandmenge kg/ha | Schutzwirkung in % |
|---|---|---|---|
| 1 | 2 g | 3 | 37.5 |

Beispiel 26

Versuch mit Antidote und Herbizid in Soja.
Applikation von Antidote und Herbizid als Tankmischung im Vorauflauf.

Blumentöpfe (oberer Durchmesser 6 cm) werden mit sandiger Lehmerde gefüllt und Sojasamen der Sorte "Hark" werden eingesät. Nach dem Bedecken der Samen wird die als Antidote zu prüfende Substanz zusammen mit dem Herbizid in verdünnte Lösung als Tankmischung auf die Bodenoberfläche versprüht. 21 Tage nach der Herbizidapplikation wird die Schutzwirkung des Antidots in Prozent bonitiert. Als Referenzen dienen dabei die mit Herbizid allein behandelten Pflanzen (keine Schutzwirkung) sowie die vollständig unbehandelte Kontrolle (100% Wachstum). Das Ergebnis ist wie folgt:

*Herbizid:* 4-Amino-6-tert.butyl-4,5-dihydro-3-methylthio-1,2,4-triazin-S-on, "Metribuzin"

| Antidote Verbindung Nr. | Aufwandmenge kg/ha | Herbizid Aufwandmenge kg/ha | Schutzwirkung in % |
|---|---|---|---|
| 1 | 0.5 | 0.5 | 25 |

Beispiel 27

Versuch mit Antidote und Herbizid in Hirse (Sorghum).
Applikation von Antidote und Herbizid als Tankmischung im Vorauflauf.

Blumentöpfe (oberer Durchmesser 6 cm) werden mit sandiger Lehmerde gefüllt und Hirsesamen der Sorte "Funk G 522" werden eingesät. Nach dem Bedecken des Samens wird die als Antidote zu prüfende Substanz zusammen mit dem Herbizid in verdünnter Lösung als Tankmischung auf die Bodenoberfläche versprüht. 14 Tage nach der Herbizidapplikation wird die Schutzwirkung des Antidots in Prozent bonitiert. Als Referenzen dienen dabei die mit Herbizid allein behandelten Pflanzen (keine Schutzwirkung) sowie die vollständig unbehandelte Kontrolle (100% Wachstum). Das Ergebnis ist wie folgt:

*Herbizid:* 2-Chlor-6-äthyl-N-(2"-methoxy-1"-methyläthyl)acet-o-toluidid, "Metolachlor"

| Antidote Verbindung Nr. | Aufwandmenge kg/ha | Herbizid Aufwandmengen kg/ha | Schutzwirkung in % |
|---|---|---|---|
| 341 | 1.5 | 1.5 | 37.5 |

Beispiel 28

Versuch mit Antidote und Herbizid in Weizen.
Applikation von Antidote und Herbizid als Tankmischung im Vorauflauf.

Weizensamen werden in Plastiktöpfe, die 0.5 l Gartenerde enthalten, im Gewächshaus ausgesät. Nach dem Auflaufen werden die Pflanzen im 2—3 Blattstadium mit den als Antidote zu prüfenden Substanzen zusammen mit dem Herbizid als Tankmischung appliziert. 20 Tage nach der Applikation wird die Schutzwirkung des Antidots in Prozent bonitiert. Als Referenzen dienen dabei die mit Herbizid allein behandelten Pflanzen (keine Schutzwirkung) sowie die vollständig unbehandelte Kontrolle (100% Wachstum). Das Ergebnis ist wie folgt:

*Herbizid:* α-[4-(3',5'-Dichlorpyridyloxy-2')-phenoxy]-propionsäure(propinyl)ester

| Antidote Verbindung Nr. | Aufwandmenge kg/ha | Herbizid Aufwandmengen kg/ha | Schutzwirkung in % |
|---|---|---|---|
| 6 | 0.5 | 0.5 | 50 |
| 71 | 0.5 | 0.5 | 25 |
| 110 | 0.5 | 0.5 | 25 |
| 334 | 0.5 | 0.5 | 37.5 |

Beispiel 29

Versuch mit Antidote und Herbizid in Getreide.
Applikation von Antidote und Herbizid als Tankmischung im Vorauflauf.

Weizen- oder Gerstensamen werden in Plastiktöpfe (oberer Durchmesser 11 cm), die 0,5 l Gartenerde enthalten, im Gewächshaus ausgesät. Nach dem Bedecken der Samen wird die als Antidote zu prüfende Substanz zusammen mit dem Herbizid als Tankmischung appliziert. 24 Tage nach der Applikation wird die Schutzwirkung des Antidots in Prozent bonitiert. Als Referenzen dienen dabei die mit Herbizid allein behandelten Pflanzen (keine Schutzwirkung) sowie die vollständig unbehandelte Kontrolle (100% Wachstum). Die Ergebnisse sind wie folgt:

*Herbizid:* 2-Chlor-6'-äthyl-N-(2"-methoxy-1"-methyläthyl)acet-o-toluidid, "Metolachlor"

Kultur Weizen (Sorte Farnese)

| Antidote Verbindung Nr. | Aufwandmenge kg/ha | Herbizid Aufwandmenge kg/ha | Schutzwirkung in % |
|---|---|---|---|
| 2 | 1 | 1 | 25 |

Kultur Gerste

| Antidote Verbindung Nr. | Aufwandmenge kg/ha | Herbizid Aufwandmenge kg/ha | Schutzwirkung in % |
|---|---|---|---|
| 1 | 1 | 1 | 25 |

Beispiel 30

Versuch mit Antidote und Herbizid in Mais.

Applikation von Antidote und Herbizid als Tankmischung im Vorauflauf.

Maissamen der Sorte "LG 5" werden in Plastiktöpfe (oberer Durchmesser 11 cm), die 0,5 l Gartenerde enthalten, im Gewächshaus ausgesät. Nach dem Bedecken der Samen wird die als Antidote zu prüfende Substanz zusammen mit dem Herbizid als Tankmischung auf die Bodenoberfläche appliziert. 18 Tage nach der Applikation wird die Schutzwirkung des Antidots in Prozent bonitiert. Als Referenzen dienen dabei die mit Herbizid allein behandelten Pflanzen (keine Schutzwirkung) sowie die vollständig unbehandelte Kontrolle (100% Wachstum). Das Ergebnis ist wie folgt:

*Herbizid:* 2-Chlor-2',6'-dimethyl-N-(2"-methoxy-1"-methyläthyl)acetanilid

| Antidote Verbindung Nr. | Aufwandmenge kg/ha | Herbizid Aufwandmenge kg/ha | Schutzwirkung in % |
|---|---|---|---|
| 2 | 2 | 2 | 25 |

Beispiel 31

Versuch mit Antidote und Herbizid in Mais.

Applikation der Antidote durch Saatbeizung.

Maissamen der Sorte "LG 5" werden mit der als Antidote zu prüfenden Substanz in einen Glasbehälter gemischt. Samen und Produkt werden durch Schütteln und Rotation gut zusammengemischt. Plastiktöpfe (oberer Durchmesser 11 cm) werden mit Gartenerde gefüllt und die gebeizten Samen werden eingesät. Nach dem Bedecken der Samen wird das Herbizid im Vorauflauf appliziert. 18 Tage nach der Herbizidapplikation wird die Schutzwirkung des Antidots in Prozent bonitiert. Als Referenzen dienen dabei die mit Herbizid allein behandelten Pflanzen (keine Schutzwirkung) sowie die vollständig unbehandelte Kontrolle (100% Wachstum). Das Ergebnis ist wie folgt:

*Herbizid:* 2-Chlor-2',6'-dimethyl-N-(methoxyäthyl)acetanilid "Dimetolachlor"

| Antidote Verbindung Nr. | Aufwandmenge kg/ha | Herbizid Aufwandmenge kg/ha | Schutzwirkung in % |
|---|---|---|---|
| 2 | 2 | 0.5 | 25 |

**Patentansprüche**

1. Verfahren zum Schützen von kulturpflanzen vor der phytotoxischen Wirkung von Herbiziden, dadurch gekennzeichnet, dass man die Kulturpflanze oder deren Samen mit einer ausreichenden Menge eines Phenylpyrimidins der Formel I behandelt

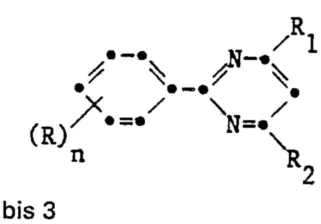

(I)

worin

n Null oder eine Zahl von 1 bis 3

R Halogen, Nitro, Cyan, —$XR_4$, —$NR_5R_6$, —$COR_3$, —$COOR_3$, $CONR_5R_6$, —CS—$NR_5R_6$, —$CH_2PO(OC_2H_5)_2$, —$SO_2$—$NR_6R_7$, —$SO_3H$, oder eine unsubstituierte oder durch Halogen, —$XR_4$, —$NR_5R_6$, —$COR_3$, —$COOR_3$, —$COR_5R_6$ oder Cyan substituierte $C_1$—$C_6$-Alkyl- oder $C_3$—$C_6$-Cycloalkylgruppe oder eine unsubstituierte oder durch Halogen oder —$XR_3$ substituierte $C_5$—$C_6$-Alkenyl-, $C_3$—$C_6$-Cycloalkenyl- oder $C_3$—$C_6$-Alkinylgruppe,

$R_1$ und $R_2$ jeweils Halogen,

$R_3$ Wasserstoff, eine unsubstituierte oder durch Halogen, —$COR_3$, —$CONR_5R_6$, Hydroxy, $C_1$—$C_6$-Alkoxy oder —$NR_5R_6$ substituierte $C_1$—$C_6$-Alkyl- oder $C_3$—$C_6$-Alkenyl- oder $C_3$—$C_6$-Alkinylgruppe,

$R_4$ dasselbe wie $R_3$, ausserdem $C_1$—$C_6$-Alkylcarbonyl, $C_3$—$C_6$-Alkenylcarbonyl, $C_3$—$C_6$-Alkinylcarbonyl,

$R_5$ und $R_6$ unabhängig voneinander je Wasserstoff, $C_3$—$C_6$-Alkenyl, $C_3$—$C_6$-Alkinyl, $C_3$—$C_6$-Cycloalkyl, oder unsubstituiertes oder durch —$COR_3$ substituiertes $C_1$—$C_6$-Alkyl, oder einer der Reste $R_5$ und $R_6$ bedeutet eine Gruppe —$OR_4$ oder —$COR_3$, —$COOR_3$, $CONR_5R_6$ und X Sauerstoff, Schwefel, —SO— oder —$SO_2$— bedeuten.

2. Verfahren gemäss Anspruch 1, dadurch gekennzeichnet, dass im Phenylpyrimidin der Formel I n sowie $R_1$ und $R_2$ wie in Anspruch 1 definiert sind und R halogen, Cyan, Nitro, Hydroxy oder Mercapto, eine $C_1$—$C_6$-Alkyl-, $C_1$—$C_6$-Alkoxy oder $C_1$—$C_6$-Alkylthiogruppe, die unsubstituiert oder durch Halogen substituiert ist, eine $C_2$—$C_6$-Alkenyl-, $C_2$—$C_6$-Alkinyl oder $C_3$—$C_6$-Cycloalkylgruppe, der Phenyl-, Carboxyl- oder Formylrest oder Aminorest, Carbamoylrest, eine $C_1$—$C_6$-Alkylcarbonyl-, $C_1$—$C_6$-Alkylamino-, Di($C_1$—$C_6$-Alkyl)amino-, Di($C_1$—$C_6$-Alkyl)carbamoylgruppe oder zwei benachbarte Reste bilden eine $C_1$—$C_6$-Alkylendioxygruppe, bedeutet.

3. Mittel zum selektiven Bekämpfen von mono- und dikotylenen Unkräutern in Nutzpflanzenkulturen, welches neben inerten Zutaten und dem Herbizid zur Erhöhung der Toleranz der Kultur eine nichtphytotoxische Menge eines Phenylpyrimidins der Formel I gemäss Anspruch 1 enthält.

4. Mittel gemäss Anspruch 3 zum selektiven Bekämpfen von mono- und dikotylenen Unkräutern in Nutzpflanzenkulturen, welches neben inerten Zutaten ein Herbizid ausgewählt aus den Klassen Chloracetanilide, Carbamate und Thiocarbamate, Diphenyläther und Nitrodiphenyläther, Benzoesäurederivate, Triazine und Triazinone, Nitroaniline, Pyridyloxyphenoxyderivate, Phosphorsäurederivate, zur Erhöhung der Toleranz der Kultur eine nicht phytotoxische Menge eines Phenylpyrimidins der Formel I gemäss Anspruch 1 enthält.

5. Verfahren zum selektiven Bekämpfen von mono- und dikotylen Unkräutern in Nutzpflanzenkulturen, dadurch gekennzeichnet, dass man die Kulturen, deren Anbaufläche oder deren Samen sowohl mit einem Herbizid als auch mit der genügenden Menge eines Phenylpyrimidins der Formel I, Anspruch 1 als Gegenmittel behandelt.

6. Verfahren gemäss Anspruch 5 zum selektiven Bekämpfen von mono- und dikotylen Unkräutern in Kulturen von Getreiden, Reis, Mais, Hirse und Soja, dadurch gekennzeichnet, dass man die Kulturen, deren Anbaufläche oder deren Samen mit einem Chloracetanilid-Herbizid und einer genügenden Menge eines Phenylpyrimidins der Formel I, Anspruch 1, als Gegenmittel behandelt.

7. Verfahren gemäss Anspruch 5 zum selektiven Bekämpfen von mono- und dikotylen Unkräutern in Kulturen von Getreiden, Reis, Mais, Hirse und Soja, dadurch gekennzeichnet, dass man die Kulturen, deren Anbaufläche oder deren Samen mit einem Carbamat-, Thiocarbamat- oder Sulfinylcarbamat-Herbizid und einer genügenden Menge eines Phenylpyrimidins der Formel I, Anspruch 1, als Gegenmittel behandelt.

8. Verfahren gemäss Anspruch 5 zum selektiven Bekämpfen von mono- und dikotylen Unkräutern in Kulturen von Getreide, Reis, Mais, Hirse und Soja, dadurch gekennzeichnet, dass man die Kulturen, deren Anbaufläche oder deren Samen mit einem Diphenyläther- oder Nitrophenyläther-Herbizid und einer genügenden Menge eines Phenylpyrimidins der Formel I, Anspruch 1, als Gegenmittel behandelt.

9. Verfahren gemäss Anspruch 5 zum selektiven Bekämpfen von mono- und dikotylen Unkräutern in Kulturen von Getreide, Reis, Mais, Hirse und Soja, dadurch gekennzeichnet, dass man die Kulturen, deren Anbaufläche oder deren Samen mit einem Benzoesäure-Derivat als Herbizid und einer genügenden Menge eines Phenylpyrimidins der Formel I, Anspruch 1, als Gegenmittel behandelt.

10. Verfahren gemäss Anspruch 5 zum selektiven Bekämpfen von mono- und dikotylen Unkräutern in Kulturen von Getreide, Reis, Mais, Hirse und Soja, dadurch gekennzeichnet, dass man die Kulturen, deren Anbaufläche oder deren Samen mit einem Triazin- oder Triazinon-Herbizid und einer genügenden Menge eines Phenylpyrimidins der Formel I, Anspruch 1, als Gegenmittel behandelt.

11. Verfahren gemäss Anspruch 5 zum selektiven Bekämpfen von mono- und dikotylen Unkräutern in Kulturen von Getreide, Reis, Mais, Hirse und Soja, dadurch gekennzeichnet, dass man die Kulturen, deren Anbaufläche oder deren Samen mit einem Nitroanilin-Herbizid und einer genügenden Menge eines Phenylpyrimidins der Formel I, Anspruch 1, als Gegenmittel behandelt.

12. Verfahren gemäss Anspruch 5 zum selektiven Bekämpfen von mono- und dikotylen Unkräutern in Kulturen von Getreide, Reis, Mais, Hirse und Soja, dadurch gekennzeichnet, dass man die Kulturen, deren Anbaufläche oder deren Samen mit einem Oxadiazolon-Herbizid und einer genügenden Menge eines Phenylpyrimidins der Formel I, Anspruch 1, als Gegenmittel behandelt.

13. Verfahren gemäss Anspruch 5 zum selektiven Bekämpfen von mono- und dikotylen Unkräutern in Kulturen von Getreide, Reis, Mais, Hirse und Soja, dadurch gekennzeichnet, dass man die Kulturen, deren Anbaufläche oder deren Samen mit einem Pyridyloxy-phenoxy-Derivat als Herbizid und einer genügenden Menge eines Phenylpyrimidins der Formel I, Anspruch 1, als Gegenmittel behandelt.

14. Verfahren gemäss Anspruch 5 zum selektiven Bekämpfen von mono- und dikotylen Unkräutern in Kulturen von Getreide, Reis, Mais, Hirse und Soja, dadurch gekennzeichnet, dass man die Kulturen, deren Anbaufläche oder deren Samen mit einem Phosphorsäure-Derivat als Herbizid und einer genügenden Menge eines Phenylpyrimidins der Formel I, Anspruch 1, als Gegenmittel behandelt.

15. Verfahren gemäss Anspruch 5 zum selektiven Bekämpfen von mono- und dikotylen Unkräutern in Kulturen von Getreide, Reis, Mais, Hirse und Soja, dadurch gekennzeichnet, dass man deren Anbaufläche oder ihre Samen mit einem Herbizid und einer genügenden Menge 2-Phenyl-4,6-dichlorpyrimidin gemäss Anspruch 1 als Gegenmittel behandelt.

16. Verfahren gemäss Anspruch 5 zum selektiven Bekämpfen von mono- und dikotylen Unkräutern in Kulturen von Getreide, Reis, Mais, Hirse und Soja, dadurch gekennzeichnet, dass man deren Anbaufläche oder ihre Samen mit einem Chloracetanilid-Herbizid und einer genügenden Menge 2-Phenyl-4,6-dichlorpyrimidin gemäss Anspruch 1 als Gegenmittel behandelt.

17. Verfahren gemäss Anspruch 5 zum selektiven Bekämpfen von mono- und dikotylen Unkräutern in Kulturen von Reis, dadurch gekennzeichnet, dass man den Reis, dessen Anbaufläche oder seine Samen mit einem Chloracetanilid-Herbizid und einer genügenden Menge 2-(4'-Tolyl)-4,6-dichloropyrimidin gemäss Anspruch 1 als Gegenmittel behandelt.

18. Verfahren gemäss Anspruch 5 zum selektiven Bekämpfen von mono- und dikotylen Unkräutern in Kulturen von Reis, dadurch gekennzeichnet, dass man den Reis, dessen Anbaufläche oder seine Samen mit einem Chloracetanilid-Herbizid und einer genügenden Menge 2-Phenyl-4,6-dibromo-pyrimidin gemäss Anspruch 1 als Gegenmittel behandelt.

19. Verfahren gemäss Anspruch 5 zum selektiven Bekämpfen von mono- und dikotylen Unkräutern in Kulturen von Reis, dadurch gekennzeichnet, dass man den Reis, dessen Anbaufläche oder seine Samen mit einem Chloracetanilid-Herbizid und einer genügenden Menge 2-(2'-Tolyl)-4,6-dibromopyrimidin gemäss Anspruch 1 behandelt.

20. Verfahren gemäss Anspruch 5 zum selektiven Bekämpfen von mono- und dikotylen Unkräutern in Kulturen von Reis, dadurch gekennzeichnet, dass man den Reis, dessen Anbaufläche oder seine Samen mit einer genügenden Menge 2-(4'-Chlorphenyl)-4,6-dichloropyrimidin gemäss Anspruch 1 behandelt.

21. Verfahren gemäss Anspruch 5 zum selektiven Bekämpfen von mono- und dikotylen Unkräutern in Kulturen von Reis, dadurch gekennzeichnet, dass man den Reis, dessen Anbaufläche oder seine Samen mit einer genügenden Menge 2-(4'-Methoxyphenyl)-4,6-dichloropyrimidin gemäss Anspruch 1 behandelt.

22. Verfahren gemäss Anspruch 5, dadurch gekennzeichnet, dass man als Herbizid 2-Chlor-2',6'-diäthyl-N-(2''-propyloxyäthyl)acetanilid verwendet.

23. Verfahren gemäss Anspruch 5, dadurch gekennzeichnet, dass man als Herbizid 2-Chlor-2',6'-diäthyl-N-(butoxymethyl)acetanilid verwendet.

24. Verfahren gemäss Anspruch 5, dadurch gekennzeichnet, dass man als Herbizid 2-Chlor-2',6'-diäthyl-N-(methoxymethyl)acetanilid verwendet.

25. Verfahren gemäss Anspruch 5, dadurch gekennzeichnet, dass man als Herbizid 2-Chlor-6'-äthyl-N-(2''-methoxy-1''-methyläthyl)accet-o-toluidid verwendet.

26. Verfahren gemäss Anspruch 5, dadurch gekennzeichnet, dass man als Herbizid 2-Chlor-6'-äthyl-N-(2'-propoxy)-1-(methyläthyl)-acet-o-toluidid verwendet.

27. Verfahren gemäss Anspruch 5, dadurch gekennzeichnet, dass man als Herbizid 2-Chlor-6'-äthyl-N-(2-butoxy-1-methyläthyl)acet-o-toluidid verwendet.

28. Verfahren gemäss Anspruch 5, dadurch gekennzeichnet, dass man das Herbizid S-2-methylpiperidino-carbonylmethyl-0,0-dipropylphosphonodithioat verwendet.

29. Verfahren gemäss Anspruch 5, dadurch gekennzeichnet, dass man als Herbizid S-4-Chlorbenzyl-diäthylthiocarbamat verwendet.

30. Verfahren gemäss Anspruch 5, dadurch gekennzeichnet, dass man als Herbizid S-4-Benzyl-diäthylthiocarbamat verwendet.

31. Mittel gemäss Anspruch 3 zum selektiven Bekämpfen von mono- und dikotylen Unkräutern in Nutzpflanzenkulturen, dadurch gekennzeichnet, dass es als Wirkstoff-Komponente ein Herbizid und ein Phenylpyrimidin der Formel 1, Anspruch 1, als Gegenmittel im Verhältnis 100:1 bis 1:5 enthält zusammen mit einem geeigneten Trägermaterial.

32. Verfahren gemäss Anspruch 1 zum Schützen von Kulturpflanzen vor der Wirkung von preemergent applizierten Herbiziden, dadurch gekennzeichnet, dass man das Saatgut der Kulturpflanzen mit einer wirksamen Menge eines 2-Phenylpyrimidins der Formel I, Anspruch 1, behandelt.

33. Saatgut von Nutzpflanzen, welches mit einer wirksamen Menge eines 2-Phenylpyrimidins der Formel I, Anspruch 1, behandelt worden ist.

34. Mittel gemäss Anspruch 4, dadurch gekennzeichnet, dass es neben inerten Zutaten ein Chloracetanilid-Herbizid und als Gegenmittel 2-Phenyl-4,6-dichlorpyrimidin enthält.

35. Mittel gemäss Anspruch 4, dadurch gekennzeichnet, dass es neben inerten inerten Zutaten 2-Chlor-2',6'-diäthyl-N-(2''-propoxyäthyl)-acetanilid als Herbizid und 2-Phenyl-4,6-dichlorpyrimidin als Gegenmittel enthält.

36. Mittel gemäss Anspruch 4, dadurch gekennzeichnet, dass es neben inerten Zutaten 2-Chlor-6'-äthyl-N-(2''-methoxy-1''-methyläthyl)acet-o-toluidid als Herbizid und 2-Phenyl-4,6-dichlorpyrimidin als Gegenmittel enthält.

## Revendications

1. Procédé pour protéger les végétaux cultivés contre les effets phytotoxiques des herbicides, caractérisé en ce que l'on traite la plante cultivée ou ses graines par une quantité suffisante d'une phénylpyrimidine de formule I:

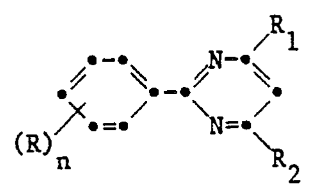

(I)

dans laquelle:

n est égal à 0 ou est un nombre de 1 à 3,

R représente un halogène, un groupe nitro, cyano, $-XR_4$, $-NR_5R_6$, $-COR_3$, $-COOR_3$, $-CONR_5R_6$, $-CS-NR_5R_6$, $-CH_2PO(OC_2H_5)_2$, $-SO_2-NR_6R_7$, $-SO_3H$, ou un groupe alkyle en $C_1-C_6$ ou cycloalkyle en $C_3-C_6$ non substitué ou substitué par des halogènes, des groupes $-XR_4$, $-NR_5R_6$, $-COR_3$, $-COOR_3$, $-COR_5R_6$ ou cyano ou un groupe alcényle en $C_5-C_6$, cycloalcényle en $C_3-C_6$ ou alcynyle en $C_3-C_6$ non substitué ou substitué par des halogènes ou des groupes $-XR_3$,

$R_1$ et $R_2$ représentent chacun un halogène,

$R_3$ représente l'hydrogène, un groupe alkyle en $C_1-C_6$ ou alcényle en $C_3-C_6$ ou alcynyle en $C_3-C_6$ non substitué ou substitué par des halogènes, desgroupes $-COR_3$, $-CONR_5R_6$, hydroxy, alcoxy en $C_1-C_6$ ou $-NR_5R_6$,

$R_4$ a les mêmes significations que $R_3$ et peut en outre représenter un groupe (alkyle en $C_1-C_6$)-carbonyle, (alcényle en $C_3-C_6$)-carbonyle, (alcynyle en $C_3-C_6$)-carbonyle,

$R_5$ et $R_6$ représentent chacun indépendamment l'un de l'autre, l'hydrogène, un groupe alcényle en $C_3-C_6$, alcynyle en $C_3-C_6$, cycloalkyle en $C_3-C_6$ ou un groupe alkyle en $C_1-C_6$ non substitué ou substitué par un groupe $-COR_3$, ou bien l'un des synboles $R_5$ et $R_6$ représente un groupe $-OR_4$ ou $-COR_3$, $-$ $COOR_3$, $CONR_5R_6$ et

X représente l'oxygène, le soufre, un groupe $-SO-$ ou $-SO_2-$.

2. Procédé selon la revendication 1, caractérisé en ce que, dans la phénylpyrimidine de la formule I:

n, $R_1$ et $R_2$ ont les significations indiquées dans la revendication 1, et

R représente un halogène, un groupe cyano, nitro, hydroxy ou mercapto, un groupe alkyle en $C_1-C_6$, alcoxy en $C_1-C_6$ ou alkylthio en $C_1-C_6$ non substitué ou substitué par des halogènes, un groupe alcényle en $C_2-C_6$, alcynyle en $C_2-C_6$ ou cycloalkyle en $C_3-C_6$, le groupe phényle, le groupe carboxyle ou le groupe formyle ou un groupe amino, un groupe carbamoyle, un groupe (alkyle en $C_1-C_6$)-carbonyle, alkylamino en $C_1-C_6$, di-(alkyle en $C_1-C_6$)-amino, di-(alkyle en $C_1-C_6$)-carbamoyle ou bien deux symboles voisins représentent ensemble un groupe alkylène-dioxy en $C_1-C_6$.

3. Produit pour combattre sélectivement les mauvaises herbes mono- et di-cotylédones dans les cultures de végétaux utiles, contenant, avec des additifs inertes et l'herbicide, et en vue d'accroître la tolérance de la culture, une quantité non phytotoxique d'une phénylpyrimidine de formule I selon la revendication 1.

4. Produit selon la revendication 3, pour combattre sélectivement les mauvaises herbes mono- et dicotylédones dans les cultures de végétaux utiles contenant, avec des additifs inertes, un herbicide choisi dans les classes des chloracétanilides, des carbamates et des thiocarbamates, des éthers diphényliques et des éthers nitrodiphényliques, des dérivés de l'acide benzoïque, des triazines et des triazinones, des nitranilines, des dérivés pyridyloxyphénoxyliques, des dérivés de l'acide phosphorique, et en vue d'accroître la tolérance de la culture, une quantité, non phytotoxique d'une phénylpyrimidine de formule I selon la revendication 1.

5. Procédé pour combattre sélectivement les mauvaises herbes mono- et di-cotylétones dans les cultures de végétaux utiles, caractérisé en ce que l'on traite les cultures, leur aire de culture ou leurs graines à la fois avec un herbicide et avec la quantité suffisante d'une phénylpyrimidine de formule I selon la revendication 1, en tant qu'antidote.

6. Procédé selon la revendication 5, pour combattre sélectivement les mauvaises herbes mono- et dicotylédones dans les cultures de céréales, de riz, de maïs, de millet et de soja, caractérisé en ce que l'on traite les cultures, leur aire de culture ou leurs graines par un herbicide du type chloracétanilide et une quantité suffisante d'une phénylpyrimidine de formule I selon la revendication 1, en tant qu'antidote.

7. Procédé selon la revendication 5, pour combattre sélectivement les mauvaises herbes mono- et dicotylédones dans les cultures de céréales, de riz, de Maïs, de millet et de soja, caractérisé en ce que l'on traite les cultures, leur aire de culture ou leurs graines par un herbicide du type carbamate, thiocarbamate ou sulfinylcarbamate et une quantité suffisante d'une phénylpyrimidine de formule I selon la revendication 1, en tant qu'antidote.

8. Procédé selon la revendication 5, pour combattre sélectivement les mauvaises herbes mono- et dicotylédones dans les cultures de céréales, de riz, de maïs, de millet et de soja, caractérisé en ce que l'on traite les cultures, leur aire de culture ou leurs graines par un herbicide du type éther diphénylique ou éther nitrophénylique et une quantité suffisante d'une phénylpyrimidine de formule I selon la revendication 1, en tant qu'antidote.

9. Procédé selon la revendication 5, pour combattre sélectivement les mauvaises herbes mono- et dicotylédones dans les cultures de céréales, de riz, de maïs, de millet et de soja, caractérisé en ce que l'on traite les cultures, leur aire de culture ou leurs graines par un dérivés de l'acide benzoïvque en tant qu'herbicide et une quantité suffisante d'une phénylpyrimidine de formule I selon la revendication 1, en tant qu'antidote.

10. Procédé selon la revendication 5, pour combattre sélectivement les mauvaises herbes mono- et dicotylédones dans les cultures de céréales, de riz, de maïs, de millet et de soja, caractérisé en ce que l'on traite les plantes cultivées, leur aire de culture ou leurs graines par un herbicide du type triazine ou triazinone et une quantité suffisante d'une phénylpyrimidine de formule I selon la revendication 1, en tant qu'antidote.

11. Procédé selon la revendication 5, pour combattre sélectivement les mauvaises herbes mono- et dicotylédones dans les cultures de céréales, de riz, de maïs, de millet et de soja, caractérisé en ce que l'on traite les plantes cultivées, leur aire de culture ou leurs graines par un herbicide du type nitraniline et une quantité suffisante d'une phénylpyrimidine de formule I selon la revendication 1, en tant qu'antidote.

12. Procédé selon la revendication 5, pour combattre sélectivement les mauvaises herbes mono- et dicotylédones dans les cultures de céréales, de riz, de maïs, de millet et de soja, caractérisé en ce que l'on traite les plantes cultivées, leur aire de culture ou leurs graines par un herbicide du type oxadiazolone et une quantité suffisante d'une phénylpyrimidine de formule I selon la revendication 1, en tant qu'antidote.

13. Procédé selon la revendication 5, pour combattre sélectivement les mauvaises herbes mono- et dicotylédones dans les cultures de céréales, de riz, de maïs, de millet et de soja, caractérisé en ce que l'on traite les plantes cultivées, leur aire de culture ou leurs graines par un dérivé pyridyloxyphénoxylique servant d'herbicide et une quantité suffisante d'une phénylpyrimidine de formule I selon la revendication 1, en tant qu'antidote.

14. Procédé selon la revendication 5, pour combattre sélectivement les mauvaises herbes mono- et dicotylédones dans les cultures de céréales, de riz, de maïs, de millet et de soja, caractérisé en ce que l'on traite les plantes cultivées, leur aire de culture ou leurs graines par un dérivé de l'acide phosphorique en tant qu'herbicide et une quantité suffisante d'une phénylpyrimidine de formule I selon la revendication 1, en tant qu'antidote.

15. Procédé selon la revendication 5, pour combattre sélectivement les mauvaises herbes mono- et dicotylédones dans les cultures de céréales, de riz, de maïs, de millet et de soja, caractérisé en ce que l'on traite leur aire de culture ou leurs graines par un herbicide et une quantité suffisante de 2-phényl-4,6-dichloropyrimidine selon la revendication 1, en tant qu'antidote.

16. Procédé selon la revendication 5, pour combattre sélectivement les mauvaises herbes mono- et dicotylédones dans les cultures de céréales, de riz, de maïs, de millet et de soja, caractérisé en ce que l'on traite leur aire de culture ou leurs graines par un herbicide du type chloracétanilide et une quantité suffisante de 2-phényl-4,6-dichlorpyrimidine selon la revendication 1, en tant qu'antidote.

17. Procédé selon la revendication 5, pour combattre sélectivement les mauvaises herbes mono- et dicotylédones dans les cultures de riz, caractérisé en ce que l'on traite le riz, son aire de culture ou ses graines par un herbicide du type chloracétanilide et une quantité suffisante de 2-(4'-tolyl)-4,6-dichloropyrimidine selon la revendication 1, en tant qu'antidote.

18. Procédé selon la revendication 5, pour combattre sélectivement les mauvaises herbes mono- et dicotylédones dans les cultures de riz, caractérisé en ce que l'on traite le riz, son aire de culture ou ses graines par un herbicide du type chloracétanilide et une quantité suffisante de 2-phényl-4,6-dibromopyrimidine selon la revendication 1, en tant qu'antidote.

19. Procédé selon la revendication 5, pour combattre sélectivement les mauvaises herbes mono- et dicotylédones dans les cultures de riz, caractérisé en ce que l'on traite le riz, son aire de culture ou ses graines par un herbicide du type chloracétanilide et une quantité suffisante de 2-(2'-tolyl)-4,6-dibromopyrimidine selon la revendication 1.

20. Procédé selon la revendication 5, pour combattre sélectivement les mauvaises herbes mono- et dicotylédones dans les cultures de riz, caractérisé en ce que l'on traite le riz, son aire de culture ou ses graines par un quantité suffisante de 2-(4'-chlorophényl)-4,6-dichloropyrimidine selon la revendication 1.

21. Procédé selon la revendication 5, pour combattre sélectivement les mauvaises herbes mono- et dicotylédones dans les cultures de riz, caractérisé en ce que l'on traite le riz, son aire de culture ou ses graines par une quantité suffisante de 2-(4'-méthoxyphényl)-4,6-dichloropyrimidine selon la revendication 1.

22. Procédé selon la revendication 5, caractérisé en ce que l'on utilise en tant qu'herbicide le 2-chloro-2',6'-diéthyl-N-(2''-propyloxyéthyl)-acétanilide.

23. Procédé selon la revendication 5, caractérisé en ce que l'on utilise en tant qu'herbicide le 2-chloro-2',6'-diéthyl-N-(butoxyméthyl)-acétanilide.

24. Procédé selon la revendication 5, caractérisé en ce que l'on utilise en tant qu'herbicide le 2-chloro-2',6'-diéthyl-N-(méthoxyméthyl)-acétanilide.

25. Procédé selon la revendication 5, caractérisé en ce que l'on utilise en tant qu'herbicide le 2-chloro-6'-éthyl-N-(2''-méthoxy-1''-méthyléthyl)-acéto-o-toluidide.

26. Procédé selon la revendication 5, caractérisé en ce que l'on utilise en tant qu'herbicide le 2-chloro-6'-éthyl-N-(2'-propoxy-1-méthyléthyl)-acéto-o-toluidide.

27. Procédé selon la revendication 5, caractérisé en ce que l'on utilise en tant qu'herbicide le 2-chloro-6'-éthyl-N-(2-butoxy-1-méthyléthyl)-acéto-o-toluidide.

28. Procédé selon la revendication 5, caractérisé en ce que l'on utilise en tant qu'herbicide le phosphonodithioate de S-méthylpipéridinocarbonylméthyle et d'0,0-dipropyle.

29. Procédé selon la revendication 5, caractérisé en ce que l'on utilise en tant qu'herbicide le diéthylthiocarbamate de S-4-chlorobenzyle.

30. Procédé selon la revendication 5, caractérisé en ce que l'on utilise en tant qu'herbicide le diéthylthiocarbamate de S-4-benzyle.

31. Produit selon la revendication 3 pour combattre les mauvaises herbes mono- et di-cotylédones dans les cultures de végétaux utiles, caractérisé en ce qu'il contient en tant que composants de la matière

active un herbicide et une phénylpyrimidine de formule I selon la revendication 1, en tant qu'antidote, dans des proportions relatives de 100:1 à 1:5, avec un véhicule approprié.

32. Procédé selon la revendication 1 pour protéger les végétaux cultivés contre les effets des herbicides appliqués en pré-levée, caractérisé en ce que l'on traite les semences des végétaux cultivés par une quantité efficace d'une 2-phénylpyrimidine de formule I selon la revendication 1.

33. Semences de végétaux utiles, qui ont été traitées par une quantité efficace d'une 2-phénylpyrimidine de formule I, selon la revendication 1.

34. Produit selon la revendication 4, caractérisé en ce qu'il contient, avec des additifs inertes, un herbicide du type chloracétanilide et, en tant qu'antidote, la 2-phényl-4,6-dichloropyrimidine.

35. Produit selon la revendication 4, caractérisé en ce qu'il contient, avec des additifs inertes, le 2-chloro-2',6'-diéthyl-N-(2''-propoxyéthyl)-acétanilide en tant qu'herbicide et la 2-phényl-4,6-dichloropyrimidine en tant qu'antidote.

36. Produit selon la revendication 4, caractérisé en ce qu'il contient, avec des additifs inertes, le 2-chloro-6'-éthyl-N-(2''-méthoxy-1''-méthyléthyl)-acéto-o-toluidide en tant qu'herbicide et la 2-phényl-4,6-dichloropyrimidine en tant qu'antidote.

**Claims**

1. A method of protecting cultivated plants from the phytotoxic effect of herbicides, characterised in that the cultivated plant, or seeds thereof, is/are treated with an effective amount of a phenylpyrimidine of the formula I

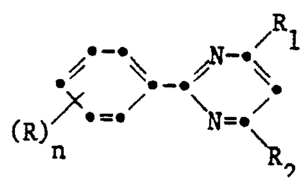

(I)

wherein

n is zero or an integer from 1 to 3,

R is halogen, nitro, cyano, $-XR_4$, $-NR_5R_6$, $-COR_3$, $-COOR_3$, $CONR_5R_6$, $-CS-NR_5R_6$, $-CH_2PO(OC_2H_5)_2$, $-SO_2-NR_6R_7$, $-SO_3H$ or a $C_1-C_6$-alkyl or $C_3-C_6$-cycloalkyl group unsubstituted or substituted by halogen, $-XR_4$, $-NR_5R_6$, $-COR_3$, $-COOR_3$, $-COR_5R_6$ or by cyano, or a $C_5-C_6$-alkenyl, $C_3-C_6$-cycloalkenyl or $C_3-C_6$-alkynyl group unsubstituted or substituted by halogen or by $-XR_3$,

$R_1$ and $R_2$ each represents halogen,

$R_3$ is hydrogen, a $C_1-C_6$-alkyl, $C_3-C_6$-alkenyl or $C_3-C_6$-alkynyl group unsubstituted or substituted by halogen, $-COR_3$, $-CONR_5R_6$, hydroxy, $C_1-C_6$ alkoxy or by $-NR_5R_6$,

$R_4$ has the same meaning as $R_3$ and is additionally $C_1-C_6$-alkylcarbonyl, $C_3-C_6$-alkenylcarbonyl or $C_3-C_6$-alkynylcarbonyl,

$R_5$ and $R_6$, each independently of the other, are hydrogen, $C_3-C_6$-alkenyl, $C_3-C_6$-alkynyl, $C_3-C_6$-cycloalkyl, or $C_1-C_6$-alkyl unsubstituted or substituted by $-COR_3$, or one of $R_5$ and $R_6$ is an $-OR_4$, $-COR_3$, $-COOR_3$ or $CONR_5R_6$ group, and

X is oxygen, sulfur, $-SO-$ or $-SO_2-$.

2. A method according to claim 1, characterised in that in the phenylpyrimidine of the formula I

n and $R_1$ and $R_2$ are as defined in claim 1 and

R is halogen, cyano, nitro, hydroxy or mercapto, a $C_1-C_6$-alkyl, $C_1-C_6$-alkoxy or $C_1-C_6$-alkythio group, each unsubstituted or substituted by halogen, a $C_2-C_6$-alkenyl, $C_2-C_6$-alkynyl or $C_3-C_6$-cycloalkyl group, the phenyl, carboxy or foryl radical or amino radical, a carbamoyl radical, a $C_1-C_6$-alkylcarbonyl, $C_1-C_6$-alkylamino, di($C_1-C_6$-alkyl)amino or di($C_1-C_6$-alkyl)carbamoyl group, or two adjacent radicals form a $C_1-C_6$-alkylenedioxy group.

3. A composition for the selective control of monocot and dicot weeds in crops of useful plants which, in addition to inert ingredients and the herbicide, contains a non-phytotoxic amount of a phenylpyrimidine of the formula I according to claim 1 in order to increase the tolerance of the crop.

4. A composition according to claim 3 for the selective control of monocot and dicot weeds in crops of useful plants which, in addition to inert ingredients and a herbicide selected from the classes chloroacetanilides, carbamates and thiocarbamates, diphenyl ethers and nitrodiphenyl ethers, benzoic acid derivatives, triazines and triazinones, nitroanilines, pyridyloxyphenoxy derivatives, and phosphoric acid derivatives, contains a non-phytotoxic amount of a phenylpyrimidine of the formula I according to claim 1 in order to increase the tolerance of the crop.

5. A method of selectively controlling monocot and dicot weeds in crops of useful plants, characterised in that the crops, the crop area or seeds of the plants are treated with both a herbicide and an effective amount of a phenylpyrimidine of the formula I, claim 1, as antidote.

6. A method according to claim 5 of the selectively controlling monocot and dicot weeds in crops of cereals, rice, maize, sorghum and soybeans, characterised in that the crops, the crop area or seeds of the

plants are treated with a chloroacetanilide herbicide and an effective amount of a phenylpyrimidine of the formula I, claim 1, as antidote.

7. A method according to claim 5 of selectively controlling monocot and dicot weeds in crops of cereals, rice, maize, sorghum and soybeans, characterised in that the crops, the crop area or seeds of the plants are treated with a carbamate, thiocarbamate or sulfinylcarbamate herbicide and an effective amount of a phenylpyrimidine of the formula I, claim 1, as antidote.

8. A method according to claim 5 of selectively controlling monocot and dicot weeds in crops of cereals, rice, maize, sorghum and soybeans, characterised in that the crops, the crop area or seeds of the plants are treated with a diphenyl ether or nitrophenyl ether herbicide and an effective amount of a phenylpyrimidine of the formula I, claim 1, as antidote.

9. A method according to claim 5 of selectively controlling monocot and dicot weeds in crops of cereals, rice, maize, sorghum and soybeans, characterised in that the crops, the crop area or seeds of the plants are treated with a benzoic acid derivative as herbicide and an effective amount of a phenylpyrimidine of the formula I, claim 1, as antidote.

10. A method according to claim 5 of selectively controlling monocot and dicot weeds in crops of cereals, rice, maize, sorghum and soybeans, characterised in that the crops, the crop area or seeds of the plants are treated with a triazine or triazinone herbicide and an effective amount of a phenylpyrimidine of the formula I, claim 1, as antidote.

11. A method according to claim 5 of selectively controlling monocot and dicot weeds in crops of cereals, rice, maize, sorghum and soybeans, characterised in that the crops, the crop area or seeds of the plants are treated with a nitroaniline herbicide and an effective amount of a phenylpyrimidine of the formula I, claim 1, as antidote.

12. A method according to claim 5 of selectively controlling monocot and dicot weeds in crops of cereals, rice, maize, sorghum and soybeans, characterised in that the crops, the crop area or seeds of the plants are treated with an oxadiazolone herbicide and an effective amount of a phenylpyrimidine of the formula I, claim 1, as antidote.

13. A method according to claim 5 of selectively controlling monocot and dicot weeds in crops of cereals, rice, maize, sorghum and soybeans, characterised in that the crops, the crop area or seeds of the plants are treated with a pyridyloxyphenoxy derivative as herbicide and an effective amount of a phenylpyrimidine of the formula I, claim 1, as antidote.

14. A method according to claim 5 of selectively controlling monocot and dicot weeds in crops of cereals, rice, maize, sorghum and soybeans, characterised in that the crops, the crop area or seeds of the plants are treated with a phosphoric acid derivative as herbicide and an effective amount of a phenylpyrimidine of the formula I, claim 1, as antidote.

15. A method according to claim 5 of selectively controlling monocot and dicot weeds in crops of cereals, rice, maize, sorghum and soybeans, characterised in that the crop area or seeds of the plants are treated with a herbicide and an effective amount of 2-phenyl-4,6-dichloropyrimidine according to claim 1 as antidote.

16. A method acording to claim 5 of selectively controlling monocot and dicot weeds in crops of cereals, rice, maize, sorghum and soybeans, characterised in that the crop area or seeds of the plants are treated with a chloroacetanilide herbicide and an effective amount of 2-phenyl-4,6-dichloropyrimidine according to claim 1 as antidote.

17. A method according to claim 5 of selectively controlling monocot and dicot weeds in rice crops, characterised in that the rice, the crop area or rice seeds are treated with a chloroacetanilide herbicide and an effective amount of 2-(4'-tolyl)-4,6-dichloropyrimidine acording to claim 1 as antidote.

18. A method according to claim 5 of selectively controlling monocot and dicot weeds in rice crops, characterised in that the rice, the crop area or rice seeds are treated with a chloroacetanilide herbicide and an effective amount of 2-phenyl-4,6-dibromopyrimidine according to claim 1 as antidote.

19. A method according to claim 5 of selectively controlling monocot and dicot weeds in rice crops, characterised in that the rice, the crop area or rice seeds are treated with a chloroacetanilide herbicide and an effective amount of 2-(2'-tolyl)-4,6-dibromopyrimidine according to claim 1.

20. A method according to claim 5 of selectively controlling monocot and dicot weeds in rice crops, characterised in that the rice, the crop area or rice seeds are treated with an effective amount of 2-(4'-chlorophenyl)-4,6-dichloropyrimidine according to claim 1.

21. A method according to claim 5 of selectively controlling monocot and dicot weeds in rice crops, characterised in that the rice, the crop area or rice seeds are treated with an effective amount of 2-(4'-methoxyphenyl)-4,6-dichloropyrimidine according to claim 1.

22. A method acording to claim 5, characterised in that the herbicide employed is 2-chloro-2',6'-diethyl-N-(2''-propoxyethyl)acetanilide.

23. A method according to claim 5, characterised in that the herbicide employed is 2-chloro-2',6'-diethyl-N-(butoxymethyl)acetanilide.

24. A method according to claim 5, characterised in that the herbicide employed is 2-chloro-2',6'-diethyl-N-(methoxymethyl)acetanilide.

25. A method according to claim 5, characterised in that the herbicide employed is 2-chloro-6'-ethyl-N-(2''-methoxy-1''-methylethyl)acet-o-toluidide.

26. A method according to claim 5, characterised in that the herbicide employed is 2-chloro-6'-ethyl-N-(2'-propoxy)-1-(methylethyl)acet-o-toluidide.

27. A method according to claim 5, characterised in that the herbicide employed is 2-chloro-6'-ethyl-N-(2-butoxy-1-methylethyl)acet-o-toluidide.

28. A method according to claim 5, characterised in that the herbicide employed is S-2-methylpiperidino-carbonylmethyl-0,0-dipropylphosphonodithioate.

29. A method according to claim 5, characterised in that the herbicide employed is S-4-chlorobenzyl-diethylthiocarbamate.

30. A method according to claim 5, characterised in that the herbicide employed is S-4-benzyl-diethylthiocarbamate.

31. A composition according to claim 3 for selectively controlling monocot and dicot weeds in crops of useful plants, characterised in that it contains, as active component, a herbicide, and a phenylpyrimidine of the formula I, claim 1, as antidote, in the ratio of 100:1 to 1:5, together with a suitable carrier therefor.

32. A method according to claim 1 of protecting cultivated plants from the effect of herbicides applied pre-emergence, characterised in that seeds of the cultivated plants are treated with an effective amount of a 2-phenylpyrimidine of the formula I, claim 1.

33. Seeds of useful plants which have been treated with an effective amount of a 2-phenylpyrimidine of the formula I, claim 1.

34. A composition according to claim 4, characterised in that in addition to inert ingredients it contains a chloroacetanilide herbicide and, as antidote, 2-phenyl-4,6-dichloropyrimidine.

35. A composition according to claim 4, characterised in that in addition to inert ingredients it contains 2-chloro-2',6'-diethyl-N-(2''-propoxyethyl)-acetanilide as herbicide and 2-phenyl-4,6-dichloropyrimidine as antidote.

36. A composition according to claim 4, characterised in that in addition to inert ingredients it contains 2-chloro-6'-ethyl-N-(2''-methoxy-1''-methylethyl)acet-o-toluidide as herbicide and 2-phenyl-4,6-dichloropyrimidine as antidote.